# EUROPEAN PATENT APPLICATION

(11) **EP 1 977 759 A1**
(43) Date of publication of application: **08.10.2008**
(21) Application number: 07105061.1
(22) Date of filing: 27.03.2007
(51) Int. Cl.: A61K 38/19, A61K 31/53, A61K 31/515, A61K 31/519, A61K 31/454, A61P 31/18

(54) **Pharmaceutical composition comprising a cytokine**

(71) Applicant: 4SC AG, 82152 Martinsried (DE)
(72) Inventor: Strobl, Stefan, 82131 Gauting (DE); Leban, Johann, Planegg-Martinsried (DE)
(74) Representative: Vossius & Partner

(57) **Abstract**

The present invention relates to a pharmaceutical composition comprising, (i) a cytokine and, (ii) a compound of the general formula (Ih) or pharmaceutically acceptable salts thereof with an acid or a base, or pharmaceutically acceptable prodrugs or a stereoisomer thereof. Exemplary cytokines are selected from the following group of cytokine families, (i) the four α-helix bundle family, which consists of, (a) the the IL-2 subfamily, (b) the interferon (IFN) subfamily and, (c) the IL-10 subfamily, (ii) the IL-1 family, (iii) the IL-17 family and, (iv) chemokines.

## Description

### Field of the Invention

The invention relates to the field of therapeutics more in particular to the treatment of diseases such as viral infections like hepatitis B or C, influenza or HIV infection, inflammatory or autoimmune diseases like multiple sclerosis, rheumatoide arthritis, inflammatory bowel disease, psoriasis, and reperfusion injuries like stroke or myocardial infarct and tumors of various kinds. The invention in particular relates to the therapeutic application of a composition comprising a cytokine and a compound according to the general formula (Ih).

### Background of the Invention

The inventors have recently shown that a compound of the general formula (Ih) or pharmaceutically acceptable salts thereof with an acid or a base, or pharmaceutically acceptable prodrugs or a stereoisomer thereof, shows unexpected, beneficial effects and can be used for the treatment of animal or human diseases.

Cytokines on the other hand are proteins which are secreted from cells, mediate and regulate immunity, inflammation, and hematopoiesis. They must be produced *de novo* in response to an immune stimulus. Responses to cytokines include increasing or decreasing expression of membrane proteins (including cytokine receptors), proliferation, and secretion of effector molecules. A number of cytokines like interferones, IL-2 or TNF-α are used as therapeutic agents in a number of diseases.

### Description of the Invention

The inventors have now even more astonishingly found that a pharmaceutical composition comprising, (i) a cytokine and, (ii) a compound of the general formula (Ih) or pharmaceutically acceptable salts thereof with an acid or a base, or pharmaceutically acceptable prodrugs or a stereoisomer thereof, shows unexpected, beneficial effects. It was found that administration of a compound of the general formular (Ih) together with a cytokine exerts unexpected synergistic effects in viral replication and PBMC proliferation assays. Even more astonishingly, the inventors can show that cytotoxic effects of both substances are diminished, thus displaying an antagonistic cytotoxic effect. Both results together broaden the applicability of both classes of substances, compounds of formular (Ih) and cytokines, if applied together by a mutual enhancement of efficacy and reduction of toxicity.

### Compounds according to the general formula (Ih)

The present invention, relates to compositions which comprise compounds of the general formula (Ih) or pharmaceutically acceptable salts thereof with an acid or a base, or pharmaceutically acceptable prodrugs or a stereoisomer thereof, 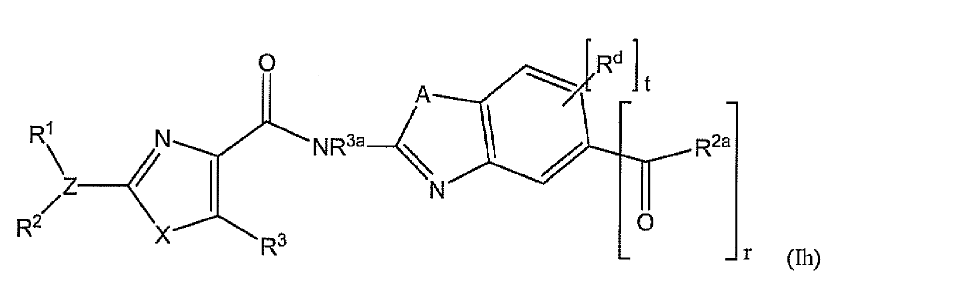 wherein
- A: is NR^{2'}, S or O;
- t: is 0 to 4;
- r: is 0,or 1;
- R^{2a}: is independently H, OH, SH, NH₂, alkyl, cycloalkyl, hydroxyalkyl, haloalkyl, haloalkyloxy, alkoxy, alkylamino, hydroxyalkylamino, halogen, aryl, or heteroaryl;
- R^{3a}: is H, OH, SH, NH₂, -C(NR⁷)NR^{7'}R⁸, -(CH₂)paryl, -(CH₂)ₚNR⁷R⁸, -C(O)NR⁷R⁸, -N=CR⁷R⁸, -NR⁷C(O)R⁸, alkyl, cycloalkyl, hydroxyalkyl, haloalkyl, haloalkyloxy, alkoxy, alkylamino, hydroxyalkylamino, halogen, aryl, or heteroaryl;
- R^{d}: is H, halogen, alkyl, -C(NR⁷)NR^{7'}R⁸, -(CH₂)paryl, -(CH₂)pNR⁷R⁸, -C(O)NR⁷R⁸, -N=CR⁷R⁸, -NR ⁷C(O)R⁸, cycloalkyl, haloalkyl, hydroxyalkyl, hydroxyalkylamino, alkylamino, heteroaryl or aryl;
- R¹: is -C(O)R^{7a}, -C(O)CHR⁷R⁸, -C(O)NR⁷R⁸, -C(O)OR⁷, -R⁷C(O)R⁸, or -C(S)R^{7b};
- R²: is H, alkyl, cycloalkyl, heterocycloalkyl, haloalkyl, hydroxyalkyl, hydroxyalkyl amino, alkylamino, or heteroaryl;
- or R¹ and R²: together with the N-atom or the C-atom to which they are attached form a 3 to 8 membered saturated or at least partially unsaturated monocyclic or polycylic ring system, wherein at least one or more of the carbon atoms in the ring is replaced by a heteroatom selected from O, N, and S, and the ring can be substituted by one or more R⁹;
- R^{4a}: is H, C₁-C₆-alkyl, C₂-C₆-alkenyl, cycloalkyl, haloalkyl, hydroxyalkyl, hydroxyalkylamino, alkylamino, -C(NR⁷)NR^{7'}R⁸, -(CH₂)paryl, -(CH₂)ₚNR⁷R⁸, -C(O)NR⁷R⁸, - N=CR⁷R⁸, -NR⁷C(O)R⁸, halogen, heteroaryl, or aryl;
- R³: is H, -C(O)NR^{a}R^{b}, halogen, alkyl, haloalkyl, aryl, heteroaryl, OH, SH, NR^{4'}OR^{5'}, NH₂, hydroxyalkylamino, alkylamino, alkoxy, cycloalkyl, heterocycloalkyl, hydroxyalkyl, or haloalkyloxy;
- R^{a}: is H, halogen, alkyl, -C(NR⁷)NR^{7'}R⁸, -(CH₂)paryl, -(CH₂)ₚNR⁷R⁸, -C(O)NR⁷R⁸, -N=CR⁷R⁸, -NR⁷C(O)R⁸, cycloalkyl, heterocycloalkyl, haloalkyl, hydroxyalkyl, hydroxyalkylamino, alkylamino, heteroaryl, or aryl;
- R^{b}: independently represents H, -CN, -OH, -SH, -CO₂R^{4'}, -C(O)R^{4'}, -SO₂NR^{4'}, -NR^{4'}R^{5'}, - C(O)NR⁷R⁸, -SO₂-alkyl, -SO₂R^{4'}, SO₃R^{4'}, -N=CR^{4'}R^{5'}, -NR^{4'}C(O)R^{4"}, -NR^{4'}-CO-haloalkyl, -NO₂, -NR4' -SO₂-haloalkyl, -NR 4' -SO₂-alkyl, -NR^{4'}-CO-alkyl, -NR^{4'}(CH₂)ₚheteroaryl, alkyl, cycloalkyl, alkylamino, alkoxy, alkylthio, halogen, haloalkyl, haloalkyloxy, -O(CH₂)ₚ[O(CH₂)ₚ]_{q}OCH₃, -C(NR^{4"})NR^{4'}benzimidazolyl, -C(NR^{4"})NR^{4'}benzthiazolyl, - C(NR^{4"})NR^{4'}benzoxazolyl, hydroxyalkyl, hydroxy-cycloalkyl, hydroxyalkylamino, heterocycloalkyl, aryl or heteroaryl;
- R⁴', R^{4"}, R^{5'}: independently are H, halogen, alkyl, -C(NR⁷)NR^{7'}R⁸, -(CH₂)ₚaryl, haloalkyl, -(CH₂)ₚNR⁷R⁸, -C(O)NR⁷R⁸, -N=CR⁷R⁸, -NR⁷C(O)R⁸, cycloalkyl, heterocycloalkyl, hydroxyalkyl, hydroxyalkylamino, alkylamino, heteroaryl, or aryl;
- R⁷, R^{7'}, R⁸: independently are H, halogen, alkyl, cycloalkyl, heterocycloalkyl, haloalkyl, hydroxyalkyl, hydroxyalkylamino, alkylamino, arylamino heteroaryl, or aryl;
- R^{7a}: is cycloalkyl, haloalkyl, hydroxyalkyl, hydroxyalkylamino, heteroaryl, or aryl;
- R^{7b}: is H, halogen, alkyl, cycloalkyl, heterocycloalkyl, haloalkyl, hydroxyalkyl, hydroxyalkyl amino, heteroaryl, or aryl;
- X: is NR^{2'}, O, or S;
- Z: is N or CR^{2'};
- R²': is H, alkyl, -C(O)NR⁷, -C(O)R ^{b}, cycloalkyl, heterocycloalkyl, haloalkyl, hydroxyalkyl, hydroxyalkylamino, alkylamino, heteroaryl, or aryl;
- p: is 1 to 6;
- q: is 1 to 6;
- R⁹: independently represents H, -CN, -OH, -SH, alkoxy, alkylthio, -CO₂R^{4'}, -C(O)R^{4a}, -C(O)NR⁷ R⁸, -SO₂NR⁴', -NR⁴'R⁵', -SO₂-alkyl, -SO₂R^{4'}, SO₃R^{4'} , -N=CR⁴'R⁵, -NR⁴C(O)R⁴", - NR⁴'-CO-haloalkyl, -NO₂, -NR^{4'}-SO₂-haloalkyl, -NR⁴'-SO₂-alkyl, -NR⁴'-CO-alkyl, -NR⁴(CH₂)pheteroaryl, alkyl, hydroxyalkyl, cycloalkyl, halogen, haloalkyl, alkylamino, -O(CH₂)ₚ[O(CH₂)ₚ]_{q}OCH₃, -C(NR^{4"})NR^{4'}benzimidazolyl, -C(NR^{4"})NR^{4'}benzthiazolyl, -C(NR^{4"})NR^{4'}benzoxazolyl, hydroxycycloalkyl, hydroxyalkylamino, haloalkyloxy, heterocycloalkyl, -(CH₂)ₚNR⁷COR⁸, aryl, or heteroaryl;
wherein
a C₁-C₆-alkyl group, if not stated otherwise, denotes a linear or branched C₁-C₆-alkyl, preferably a linear or branched chain of one to five carbon atoms, which can optionally be substituted by one or more substituents R';
a C₂-C₆-alkenyl group, if not stated otherwise, denotes a linear or branched C₂-C₆-alkenyl, preferably a linear or branched chain of two to six carbon atoms, which can optionally be substituted by one or more substituents R';
an alkyl group, if not stated otherwise, denotes a linear or branched C₁-C₆-alkyl, preferably a linear or branched chain of one to six carbon atoms, a linear or branched C₂-C₆-alkenyl or a linear or branched C₂-C₆-alkynyl group, which can be substituted by one or more substituents R';
R' is independently H, -CO₂R", -CONHR", -CR"O, -SO₂NR", -NR"-CO-haloalkyl, -NO₂, -NR"-SO₂-haloalkyl, -NR"-SO₂-alkyl, -SO₂-alkyl, -NR"-CO-alkyl, -CN, alkyl, cycloalkyl, alkylamino, alkoxy, -OH, -SH, alkylthio, hydroxyalkyl, hydroxyalkylamino, halogen, haloalkyl, haloalkyloxy, aryl, or heteroaryl;
R" is independently H, haloalkyl, hydroxyalkyl, alkyl, cycloalkyl, aryl, or heteroaryl;
a cycloalkyl group denotes a non-aromatic ring system containing three to eight carbon atoms, preferably four to eight carbon atoms, wherein one or more of the carbon atoms in the ring can be substituted by a group R' being as defined above; the C₃-C₈-cycloalkyl residue may be selected from the group comprising -cyclo-C₃H₅, -cyclo-C₄H₇, -cyclo-C₅H₉, -cyclo-C₆H₁₁,-cyclo-C₇H₁₃, -cyclo-C₈H₁₅;
a heterocycloalkyl group denotes a non-aromatic ring system containing two to ten carbon atoms and at least one heteroatom selected from O, N, and S, wherein one or more of the carbon atoms in the ring can be substituted by R' being as defined above; preferred heterocycloalkyl groups are morpholine-4-yl, piperazinyl, 1-alkylpiperazine-4-yl, piperidinyl, pyrrolidinyl, azepane-1-yl;
an alkoxy group denotes an O-alkyl group, the alkyl group being as defined above; the alkoxy group is preferably a methoxy, ethoxy, isopropoxy, t-butoxy or pentoxy group;
an alkylthio group denotes an S-alkyl group, the alkyl group being as defined above;
a haloalkyl group denotes an alkyl group which is substituted by one to five halogen atoms, the alkyl group being as defined above; the haloalkyl group is preferably a -C(R¹⁰)₃, -CR¹⁰(R^{10'})₂, -CR¹⁰(R^{10'})R^{10"} -C₂(R¹⁰)₅, -CH₂C(R¹⁰)₃, -CH₂CR¹⁰(R^{10'})₂, -CH₂CR¹⁰(R^{10'})R^{10"}, -C₃(R¹⁰)₇, or -C₂H₄C(R¹⁰)₃ wherein R¹⁰, R^{10'}, R^{10"} represent F, Cl, Br or I, preferably F;
a hydroxyalkyl group denotes an HO-alkyl group, the alkyl group being as defined above;
a haloalkyloxy group denotes an alkoxy group which is substituted by one to five halogen atoms, the alkyl group being as defined above; the haloalkyloxy group is preferably a -OC(R¹⁰)₃, -OCR¹⁰(R^{10'})₂, -OCR¹⁰(R^{10'})R^{10"}, -OC₂(R¹⁰)₅, -OCH₂C(R¹⁰)₃, -OCH₂CR¹⁰(R^{10'})₂, - OCH₂CR¹⁰(R¹⁰)R^{10"}, -OC₃(R¹⁰)₇, or -OC₂H₄C(R¹⁰)₃, wherein R¹⁰, R^{10'}, R^{10"} represent F, Cl, Br or I, preferably F;
a hydroxyalkylamino group denotes an (HO-alkyl)₂-N- group or HO-alkyl-NH- group, the alkyl group being as defined above;
an alkylamino group denotes an HN-alkyl or N-dialkyl group, the alkyl group being as defined above;
a halogen group is fluorine, chlorine, bromine, or iodine;
an aryl group denotes an aromatic group having five to fifteen carbon atoms, which can be substituted by one or more substituents R', where R' is as defined above; the aryl group is preferably a benzyl group, a phenyl group, -o-C₆H₄-R', -m-C₆H₄-R', -p-C₆H₄-R', 1-naphthyl, 2-naphthyl, 1-anthracenyl or 2-anthracenyl, R' being as defined above;
an arylamino group denotes an HN-aryl or N-diaryl group, the aryl group being as defined above;
a heteroaryl group denotes a 5- or 6-membered heterocyclic group which contains at least one heteroatom selected from O, N, and S. This heterocyclic group can be fused to another aromatic ring. For example, this group can be selected from a thiadiazole, thiazol-2-yl, thiazol-4-yl, thiazol-5-yl, isothiazol-3-yl, isothiazol-4-yl, isothiazol-5-yl, oxazol-2-yl, oxazol-4-yl, oxazol-5-yl, isooxazol-3-yl, isooxazol-4-yl, isooxazol-5-yl, benzooxazol-2-yl, benzooxazol-4-yl, benzooxazol-5-yl, benzoisooxazol-3-yl, benzoisooxazol-4-yI, benzoisooxazol-5-yl, 1,2,4-oxadiazol-3-yl, 1,2,4-oxadiazol-5-yl, 1,2,5-oxadiazol-3-yl, 1,2,5-oxadiazol-4-yl, 1,2,4-thiadiazol-3-yl, 1,2,4-thiadiazol-5-yl, isothiazol-3-yl, isothiazol-4-yl, isothiazol-5-yl, benzoisothiazol-3-yl, benzoisothiazol-4-yl, benzoisothiazol-5-yl, 1,2,5-thiadiazol-3-yl, 1-imidazolyl, 2-imidazolyl, 1,2,5-thiadiazol-4-yl, 4-imidazolyl, benzoimidazol-4-yl, 1-pyrrolyl, 2-pyrrolyl, 3-pyrrolyl, 2-furanyl, 3-furanyl, 2-thienyl, 3-thienyl, 2-pyridyl, 3-pyridyl, 4-pyridyl, 2-pyranyl, 3-pyranyl, 4-pyranyl, 2-pyrimidinyl, 4-pyrimidinyl, 5-pyrimidinyl, 6-pyrimidinyl, 2,4-dimethoxy-6-pyrimidinyl, 3-pyridazinyl, 4-pyridazinyl, 2-pyrazinyl, 1-pyrazolyl, 3-pyrazolyl, 4-pyrazolyl, 1,2,3-triazol-4-yl, 1,2,3-triazol-5-yl, 1,2,4-triazol-3-yl, 1,2,4-triazol-5-yl, 1,3,5-triazol-6-yl, 2,4-dimethoxy-1,3,5-triazol-6-yl, 1H-tetrazol-2-yl, 1H-tetrazol-3-yl, tetrazolyl, acridyl, furazane, indazolyl, phenazinyl, carbazolyl, phenoxazinyl, indolizine, 2-indolyl, 3-indolyl, 4-indolyl, 5-indolyl, 6-indolyl, 7-indolyl, 1-isoindolyl, 3-isoindolyl, 4-isoindolyl, 5-isoindolyl, 6-isoindolyl, 7-isoindolyl, 2-indolinyl, 3-indolinyl, 4-indolinyl, 5-indolinyl, 6-indolinyl, 7-indolinyl, benzo[b]furanyl, benzofurazane, benzothiofurazane, benzotriazol-1-yl, benzotriazol-4-yl, benzotriazol-5-yl, benzotriazol-6-yl, benzatriazal-7-yl, benzotriazine, benzo[b]thiophenyl, benzimidazol-2-yl, 1H-benzimidazolyl, benzimidazol-4-yl, benzimidazol-5-yl, benzimidazol-6-yl, benzimidazol-7-yl, benzothiazolyl, quinazolinyl, quinoxazolinyl, cinnoline, quinolinyl, tetrahydroquinolinyl, isoquinolinyl, tetrahydro-thieno[3,4-d]imidazol-2-one, pyrazolo[5,1-c][1,2,4]triazine, 2,3-dihydrobenzo[1,4]-dioxin-2-yl, 2,3-dihydrobenzo[1,4]-dioxin-3-yl, 2,3-dihydrobenzo[1,4]-dioxin-5-yl, 2,3-dihydrobenzo[1,4]-dioxin-6-yl, 2,6-dimethoxypyririmidin-3-yl, 2,6-dimethoxypyrimidin-4-yl, tetrahydroisoquinolinyl, purine, phthalazine, pteridine, thiatetraazaindene, thiatriazaindene, isothiazolopyrazine, isothiazolopyrimidine, pyrazolotriazine, pyrazolopyrimidine, imidazopyridazine, imidazo-pyrimidine, imidazopyridine, imidazolotriazine, triazolotriazine, triazolopyridine, triazolopyrazine, triazolopyrimidine, 4-[1,2,4]triazolo[4,3-a]pyridin-3-yl, 1-furo[2,3-c]pyridin-4-yl, 1-furo[2,3-c]pyridin-5-yl, 1-furo[2,3-c]pyridin-3-yl, and triazolopyridazine group. This heterocyclic group can be substituted by one or more substituents R', wherein R' is as defined above.

In formula (Ih), the following substituents are preferred, alone or in combination:
X is preferably S or O, more preferably S.
R^{3a} is preferably H.

In a preferred embodiment, Z is CR^{2'} and R¹ and R² together with the C-atom to which they are attached form a 6 membered saturated ring. In another preferred embodiment, Z is N and R¹ and R² together with the N-atom to which they are attached form a 6 membered saturated ring.

R⁹ is preferably heteroaryl, aryl or benzyl, more preferably heteroaryl. R⁹ is even more preferably thienopyrimidine, quinazoline, purine, pyrazolopyrimidine or triazolpyrimidine. Even more preferably, R⁹ is thienopyrimidine.
t is preferably 0.
r is preferably 1.
R^{2a} is preferably OH, alkyl, aryl or heteroaryl, more preferably alkyl, especially methyl.

A preferred embodiment of the invention, the compounds (Ih) in the composition are compounds of the formula (Iha), 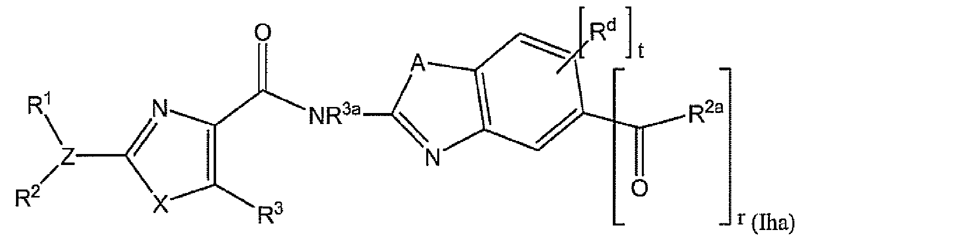 wherein
R¹ is COR^{7a};
R² and R^{3a} are H;
A is NH;
R⁷ is as defined above;
ZisN;
X is NR^{2'}, O or S;
R³ is H, methyl, ethyl, methoxy, amine, alkylamine, morpholino, N-methylpiperazine, CF₃, or
OCF₃;
t is 0;
r is 1; and
R²' is optionally substituted aryl, benzyl or heteroaryl.

In another preferred embodiment of the invention, in the compounds of formula (Ih), are compounds of the formula (Iha), wherein R^{7a} is optionally substituted aryl, benzyl or heteroaryl.

In another preferred embodiment of the invention, in the compounds of formula (1h), are compounds of the formula (Ihb), 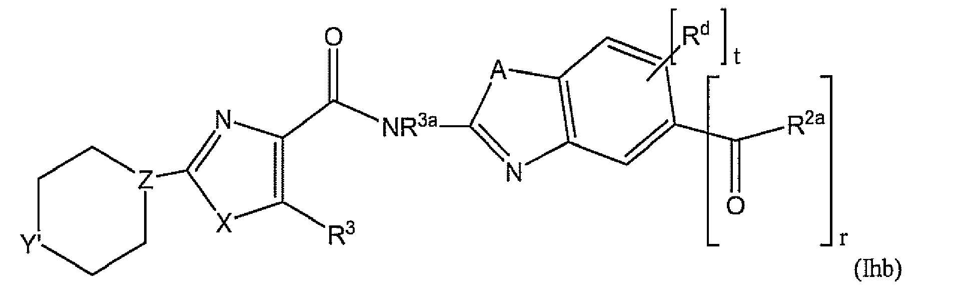 wherein
R^{3a} is H;
A is NH;
X is NR^{2'}, O or S;
Y' is O or NR^{2'};
R^{2'} is as defined above;
R³ is H, methyl, ethyl, methoxy, amine, alkylamine, morpholino, N-methylpiperazine, CF₃, or OCF₃;
t is 0;
r is 1; and
R^{2a} is optionally substituted aryl, benzyl or heteroaryl;
Z is defined as above.

In formula (Ihb), the following substituents are preferred, alone or in combination:
Z is preferably CR^{2'}.
Y' is preferably NR^{2'}. In a preferred embodiment, the R^{2'} in the NR^{2'} of Y' is preferably substituted or unsubstituted heteroaryl. In another preferred embodiment, the R^{2'} in the NR^{2'} of Y' is aryl. In yet another preferred embodiment, the R^{2'} in the NR^{2'} of Y' is benzyl. In a more prefered embodiment, the R^{2'} in the NR^{2'} of Y' is pyrimidine or triazine. In another more preferred embodiment, the R² in the NR^{2'} of Y' is a substituted or unsubstituted bicyclic heteroaryl. more preferably thienopyrimidine, quinazoline, purine, pyrazolopyrimidine or triazolpyrimidine, even more preferably thienopyrimidine.

In a preferred embodiment, X is S. In another preferred embodiment, X is O. In yet another preferred embodiment, X is NR^{2'}.
R³ is preferably H.
R^{2a} is preferably aryl or heteroaryl, more preferably phenyl.

In another more preferred embodiment of the invention, in the compounds of formula (Ih), are compounds of the formula (Ihe), 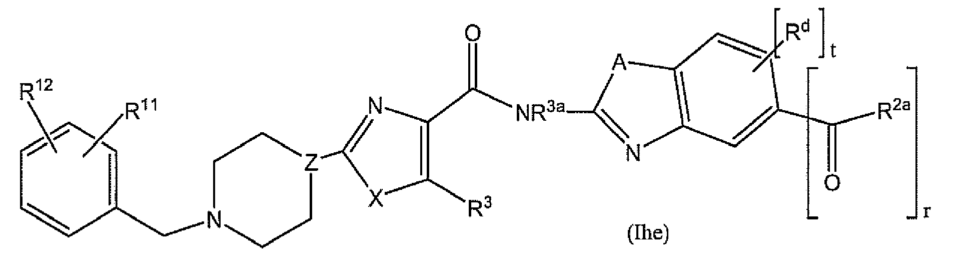 wherein
R^{3a} is H; A is NH ; X is NR^{2'}, O or S;
R¹¹ and R¹² independently represent H, -CN, -OH, -SH, -CO₂R^{4'}, -C(O)R^{4'}, -SO₂NR^{4'} , -NR^{4'}R^{5'}, -SO₂-alkyl, -SO₂R^{4'}, SO₃R^{4'}, -N=CR^{4'}R^{5'}, -NR^{4'}C(O)R^{4"}, -NR^{4'}-CO-haloalkyl, -NO₂, -NR^{4'}-SO₂-haloalkyl, -NR^{4'}-SO₂-alkyl, -NR^{4'}-CO-alkyl, -NR^{4'} (CH₂)ₚheteroaryl, alkyl, cycloalkyl, alkylamino, alkoxy, alkylthio, -O(CH₂)ₚ[O(CH₂)ₚ]_{q}OCH₃, - C(NR^{4"})NR^{4'}benzimidazolyl, -C(NR^{4"})NR^{4'}benzthiazolyl, -C(NR^{4"})NR^{4'}benzoxazolyl hydroxyalkyl, hydroxycycloalkyl, hydroxyalkylamino, halogen, haloalkyl, haloalkyloxy, aryl, arylalkyl or a heterocycle;
R^{4'}, R^{4"}, R^{5'} are defined as above;
R^{2'} is as defined above;
R³ is H, methyl, ethyl, methoxy, amine, alkylamine, morpholino, N-methylpiperazine, CF₃, or OCF₃;
t is 0;
r is 1; and
R^{2a} is optionally substituted aryl, benzyl or heteroaryl.

In formula (Ihe), the following substituents are preferred, alone or in combination:
Z is preferably CR^{2'}.

In a preferred embodiment, X is S. In another preferred embodiment, X is O. In yet another preferred embodiment, X is NR^{2'}.
R¹¹ is preferably -H, -CN, -OH, halogen, haloalkyl, haloalkyloxy, more preferably -CN.
R¹² is preferably H.
R^{2a} is preferably aryl or heteroaryl, more preferably phenyl.

For the compounds of the formula (Ih) the term "stereoisomer" means cis/trans or E/Z isomerism. More particularly, the possible double bond(s) present in the various substituent of the compounds of the present invention can be E or Z configuration. These pure or impure geometrical isomers, alone or as a mixture, form an integral part of the compounds of the formula (Ih). The term "stereoisomer" includes also all the isomeric forms, alone or as mixture, resulting from the presence of one or more axes and/or centres of symmetry in the molecules, and resulting in the rotation of a beam of polarized light. More particularly, it includes enatiomers and diastereomers, in pure form or as a mixture.

The compounds of the formula (Ih) to be used according to the invention can form salts with inorganic or organic acids or bases. Examples of pharmaceutically acceptable salts comprise without limitation non-toxic inorganic or organic salts such as acetate derived from acetic acid, aconitate derived from aconitic acid, ascorbate derived from ascorbic acid, benzoate derived from benzoic acid, cinnamate derived from cinnamic acid, citrate derived from citric acid, embonate derived from embonic acid, enantate derived from heptanoic acid, formiate derived from formic acid, fumarate derived from fumaric acid, glutamate derived from glutamic acid, glycolate derived from glycolic acid, chloride derived from hydrochloric acid, bromide derived from hydrobromic acid, lactate derived from lactic acid, maleate derived from maleic acid, malonate derived from malonic acid, mandelate derived from mandelic acid, methanesulfonate derived from methanesulfonic acid, naphtaline-2-sulfonate derived from naphtaline-2-sulfonic acid, nitrate derived from nitric acid, perchlorate derived from perchloric acid, phosphate derived from phosphoric acid, phthalate derived from phthalic acid, salicylate derived from salicylic acid, sorbate derived from sorbic acid, stearate derived from stearic acid, succinate derived from succinic acid, sulphate derived from sulphuric acid, tartrate derived from tartaric acid, toluene-p-sulfate derived from p-toluene-sulfonic acid and others. Such salts can be produced by methods known to someone of skill in the art and described in the prior art.

Other salts like oxalate derived from oxalic acid, which is not considered as pharmaceutically acceptable can be appropriate as intermediates for the production of compounds of the formula (Ih) or a pharmaceutically acceptable salt thereof or pharmaceutically acceptable prodrugs, or a stereoisomer thereof.

The invention covers the pharmaceutically acceptable salts, as indicated above, but also salts allowing a suitable seperation or crystallization of the compounds of the formula (Ih), such as the salts obtained with chiral amines.

The compounds of the formula (Ih) above also comprise the prodrugs of these compounds. The term "prodrug" as used herein refers to compounds which once administered to the patient are not pharmaceutically active themselves ('prodrugs') but which are chemically and/or biologically transformed into their pharmaceutical active form (compounds of formula (Ih)) in vivo, i.e. in the subject to which the compound is administered. Prodrugs include, for example, compounds of the invention wherein hydroxy, amine or sulfhydryl groups are bonded to any group that, when administered to a patient, cleaves to form the hydroxy, amine or sulfhydryl groups. Thus, representative examples of prodrugs include, but are not limited to, acetate, formate and benzoate derivatives of alcohol, sulfhydryl and amine functional groups of the compounds of the present invention. Further, in the case of carboxylic acid (-COOH), esters may be employed, such as methyl esters, ethyl esters, double esters and the like. Esters may be active in their own right and/or be hydrolysable under in vivo conditions in the human body.

### Cytokine according to the Invention

Cytokines are proteins which are secreted from cells and mediate and regulate immunity, inflammation, and hematopoiesis. They must be produced *de novo* in response to an immune stimulus. Responses to cytokines include increasing or decreasing expression of membrane proteins (including cytokine receptors), proliferation, and secretion of effector molecules.

The composition according to the invention comprises a cytokine which is selected from the group of (a) an autocrine cytokine, (b) a paracrine cytokine and, (c) an endocrine cytokine. Autocrine cytokines act on the cells that secret it. Paracrine cytokine action is restricted to the vicinity of the cytokine's secretion and endocrine cytokines diffuse to distant regions of the body. The composition may also comprise mor than one cytokine.

The composition according to the invention preferably comprises a cytokine wherein the cytokine is selected from the following group of cytokine families, (i) the four α-helix bundle family, which consists of, (a) the IL-2 subfamily, (b) the interferon (IFN) subfamily and, (c) the IL-10 subfamily (including several non-immunological cytokines including erythropoietin (EPO) and thrombopoietin (THPO)), (ii) the IL-1 family (e.g. IL-1 and IL-18), (iii) the IL-17 family and, (iv) chemokines.

The IL-2 family is preferred. Interleukin-2 belongs to a family of cytokines, which includes IL-4, IL-7, IL-9, IL-15 and IL-21. IL-2 signals through a receptor complex consisting of IL-2 specific IL-2 receptor alpha (CD25), IL-2 receptor beta (CD 122) and a common gamma chain (yc), which is shared by all members of this family of cytokines. Binding of IL-2 activates the Ras/MAPK, JAK/Stat and PI 3-kinase/Akt signaling modules.

The interferone family is further preferred. It includes type I interferones, type II interferones and type III interferones. Type I includes IFN-α (alpha), IFN-β (beta), IFN-κ (kappa), IFN-δ (delta), IFN-ε (epsilon), IFN-τ (tau), IFN-ω (omega) and IFN-ζ (zeta, also known as limitin). Type II includes the sole member IFN-γ (gamma). Type III interferones include IFN-λ (lambda) molecules called IFN-λ1, IFN-λ2 and IFN-λ3 (also called IL29, IL28A and IL28B respectively).Typ I and II are preferred. More preferred are IFN-α (alpha), IFN-β (beta) and IFN-γ (gamma).

Tumor-Necrosis-Factor (TNF) is further preferred. Herein, this includes TNF-α (alpha) and TNF-β (beta).

**IFN-α**
In a particularly preferred embodiment IFN-α is the cytokine in the composition. The 13 human IFN-α genes encode 12 different IFN-α proteins. The IFN-α proteins share 76-99% sequence homology and differ little in their biological activity, therefore commercial IFN-α is IFN-α1. IFN-α administered subcutaneously demonstrated a surprising wide range of efficacy in haematological malignancies including tumors of presumed B-cell, T-cell and myeloid lineages. In some diseases, as hairy cell leukaemia and chronic myelogenous leukaemia, IFN-α was broadly effective. However, at present, IFN-α has a limited role in the treatment of hairy cell leukaemia, and it is reserved for selected patients who failed nucleoside therapy.

Commercially the most important application of IFN- α is its use for the treatment of hepatitis C (HCV). Infections with HCV are common worldwide. The WHO estimates that about 3 % of the world's population are HCV carriers. The genetic heterogeneity of HCV plus the significant antigenic variation among virus quasispecies within individual patients probably explains the difficulties in the development of vaccines.

IFN-α therapy in chronic HCV infected patients achieved a sustained antiviral response only in 15-20 % of patients. A combination therapy with ribavirin obtained a sustained response of 38-43 %. An additional improvement in the treatment of HCV has been achieved with the development ofpegylated IFN-α, to improve the pharmacokinetic profile. The response rate of pegylated IFN-α is in the range of IFN-α, ribavirin cotherapy.
The current treatment is pegylated IFN-α and ribavirin. Brand names are Copegus (Hoffmann-La Roche) and Rebetol (Schering-Plough). With this treatment an average response of 50-60 % is obtained. HCV genotype 1 has only a response rate of 42-52 %.

Because of the high percentage of non responders to standard therapy, there is still a great need for improvement of HCV therapy.

In a preferred embodiment the invention relates to the use of a composition according to the invention, wherein the cytokine is IFN-α and the compound is according to the gemeal formula (Ih), for the production of a medicament for the treatment of a disease selected from the group of HCV therapy, early and late state chronic HCV infection, viral infections such as chronic hepatitis B, chronic hepatitis D, bunyamwera virus, positive-sense ssRNA Viruses, picornaviruses, flaviviruses, coronavirus, influenzavirus, avian influenza virus, human solid and haematological malignancies, hairy cell leukaemia, renal carcinoma, basal cell carcinoma, malignant myeloma, Karposi sarcoma, multiple myeloma, chronic, myelogous leukaemia, non-hodgkins lymphoma, burkitt lymphoma, follicular lymphoma, , cryptococal meningitis, polycythemia vera, essential thrombocythemia, uveitis, laryngeal papillomatosis, mycosis fungoides, condyloma acuminate, multiple sclerosis, chronic granulomatous disease, osteoporosis, infectious diseases, mycobacterial infections, leprosy, mycobacterium avium infections, tuberculosis, leishmaniasis, opportunistic infections in HIV disease, stroke, myocardial ischemia.

The following indications are preferred: HCV infection, chronic hepatitis B, chronic hepatitis D, influenza infection, HIV infection, stroke, myocardial ischemia, multiple sclerosis, osteoporosis.

The following indication is particularly preferred: HCV infection.

**IFN-β**
IFN-β is preferred in the composition. The use of IFN-β has been largely confined to multiple sclerosis (MS). MS affects approximately 2.5 million individuals worldwide. MS is twice as common in women as in men. IFN-β, is an anti-inflammatory and immunomodulatory interferon which reduces relapse frequency in multiple sclerosis (MS) and ameliorates experimental autoimmune neuritis.

Consequently, recombinant human interferon β is an FDA approved treatment for patients with relapsing remitting multiple sclerosis, in whom the safety profile is well characterized.

In a preferred embodiment the invention relates to the use of a composition according to the invention, wherein the cytokine is IFN-β and the compound is according to the general formula (Ih), for the production of a medicament for the treatment of a disease selected from the group of viral infections such as HCV, chronic hepatitis B, chronic hepatitis D, bunyamwera virus, positive-sense ssRNA Viruses, picornaviruses, flaviviruses, coronavirus, influenzavirus, avian influenza virus, human solid and haematological malignancies, hairy cell leukaemia, renal carcinoma, basal cell carcinoma, malignant myeloma, Karposi sarcoma, multiple myeloma, chronic, myelogous leukaemia, non-hodgkins lymphoma, burkitt lymphoma, follicular lymphoma, , cryptococal meningitis, polycythemia vera, essential thrombocythemia, uveitis, laryngeal papillomatosis, mycosis fungoides, condyloma acuminate, multiple sclerosis, chronic granulomatous disease, osteoporosis, infectious diseases, mycobacterial infections, leprosy, mycobacterium avium infections, tuberculosis, leishmaniasis, opportunistic infections in HIV disease, stroke, myocardial ischemia

The following indications are preferred: HCV infection, chronic hepatitis B, chronic hepatitis D, influenza infection, HIV infection, stroke, myocardial ischemia, multiple sclerosis, osteoporosis..

The following indication is particularly preferred: multiple sclerosis.

**IFN-γ**
Human IFN-γ is a preferred cytokine in the composition. It is a glycosylated protein that has minimal sequence homology with IFN-α or IFN-β IFN-γ is produced by activated T cells and natural killer cells in response to specific mitogenic stimuli. While IFN-γ shares many activities with the type I interferons, it also exhibits many other activities, which has led to the conclusion that its natural role is the modulation of the immune system. The clinical application of IFN-γ is limited to rare diseases. IFN-γ has FDA approval for chronic granulomatous disease (CGD). CDG is a rare ,co genital disorder that causes patients, mainly children, to be vulnerable to severe, recurrent bacterial and fungal infections.

The second FDA-approved clinical application is for congenial osteoporosis a life threatening paediatric disease. The most common adverse events during IFN-γ therapy are fever, headache, nausea and vomiting.

In a preferred embodiment the invention relates to the use of a composition according to the invention, wherein the cytokine is IFN-γ and the compound is according to the general formula (Ih), for the production of a medicament for the treatment of a disease selected from the group of viral infections such as HCV, chronic hepatitis B, chronic hepatitis D, bunyamwera virus, positive-sense ssRNA Viruses, picornaviruses, flaviviruses, coronavirus, influenzavirus, avian influenza virus, human solid and haematological malignancies, hairy cell leukaemia, renal carcinoma, basal cell carcinoma, malignant myeloma, Karposi sarcoma, multiple myeloma, chronic, myelogous leukaemia, non-hodgkins lymphoma, burkitt lymphoma, follicular lymphoma, , cryptococal meningitis, polycythemia vera, essential thrombocythemia, uveitis, laryngeal papillomatosis, mycosis fungoides, condyloma acuminate, multiple sclerosis, chronic granulomatous disease, osteoporosis, infectious diseases, mycobacterial infections, leprosy, mycobacterium avium infections, tuberculosis, leishmaniasis, opportunistic infections in HIV disease, stroke, myocardial ischemia

The following indications are preferred: HCV infection, chronic hepatitis B, chronic hepatitis D, influenza infection, HIV infection, stroke, myocardial ischemia, multiple sclerosis, osteoporosis, CDG.

The following indications are particularly preferred: CDG and congenial osteoporosis.

**TNF**
Tumor necrosis factor alpha (TNF) is a preferred cytokine in the composition, it was isolated 30 years ago, it is a multifunctional cytokine which plays a key role in apoptosis and cell survival as well as in inflammation and immunity. Although named for its antitumor properties, TNF has been implicated in a wide spectrum of other diseases. The current use of TNF in cancer is in the regional treatment of locally advanced soft tissue sarcomas and metastatic melanomas and other irresectable tumors of any histology to avoid amputation of the limb. It has been demonstrated in the isolated limb perfusion setting that TNF acts synergistically with cytostatic drugs. High-dose TNF-alpha plus chemotherapy, with or without IFN-γ, can be safely administered regionally through isolated limb perfusion. This procedure produced between 70% and 80% complete remission in cases of in transit melanoma metastases and between 25% and 36% complete remission in cases of inextirpable soft-tissue sarcomas. Dual targeting is involved; TNF-α and IFN-g induce apoptosis of angiogenic endothelium, while melphalan induces apoptosis of tumour cells (Curr Opin Immunol1998 Oct;10(5):573-80).

In a preferred embodiment the invention relates to the use of a composition according to the invention, wherein the cytokine is TNF and the compound is according to the general formula (Ih), for the production of a medicament for the treatment of a disease selected from the group of solid tumours and hematopoetic cancers.

The following indications are preferred: solid tumours.

The following indication is particularly preferred: soft tissue sarcomas and metastatic melanomas

**IL-2**
IL-2 is a preferred preferred cytokine in the composition. Human immunodeficiency virus type 1 (HIV-1) gene expression and transcription is an essential step in the viral life cycle, which is considered to be a possible target for inhibition of HIV-1 replication. Infection with HIV-1 leads to progressive CD4 T-cell death, resulting in AIDS development. The mechanisms that trigger this CD4 T-cell death are still not fully understood, but HIV-1-induced CD4 T-cell killing is now recognized as central to immunodeficiency. Antiretroviral therapy (ART) combined with IL-2 produced a substantially greater increase in CD4 cells and was associated with a larger decrease in viral load than ART alone (Davey RT et al., JAMA. 2000 Oct 25;284(16):2055-6).

In a preferred embodiment the invention relates to the use of a composition according to the invention, wherein the cytokine is IL-2 and the compound is according to the general formula (Ih), for the production of a medicament for the treatment of a disease selected from the group of viral infections in particular HIV infections.

In one embodiment of the invention the active substances according to the invention, i.e. the compound according to the general formula (Ih) and the cytokine, are not present in a single composition but are administered separately, but simultaneously. Herein, simultaneously according to the invention may mean that, either (i) the substances are administered separately at the same time, or, (ii) one or the other substance is administered first and the second substance is administered shortly thereafter, however in such a timely fashion that the additive and/or synergistic effect taught herein, is maintained. In a preferred embodiment of the above teaching, e.g. the cytokine may be administered as an injection whereas the compound according to the general formula (Ih) is administered in another or the same manner in any case however, separately but simultaneously wherein, simultaneously has the meaning outlined above herein.

"Treatment" according to the present invention is intended to mean complete or partial healing of a disease, or alleviation of a disease or stop of progression of a given disease.

Thus, in one embodiment, the invention relates to the use of the composition according to the invention, *i.e.* comprising a cytokine and a compound according to formula (Ih) or a pharmaceutically acceptable salt or pharmaceutically acceptable prodrugs, or a stereoisomer thereof if desired with appropriate adjuvants and additives for the production of a medicament.

The compound according to formula (Ih) may be present in the composition as a pharmaceutically acceptable salt or pharmaceutically acceptable prodrug, or a stereoisomer thereof if desired with appropriate adjuvants and additives.

Furthermore, the invention relates to a method of treatment or prevention of diseases which comprises the administration of an effective amount of the composition according to the invention comprising a cytokine and a compound (Ih) or a pharmaceutically acceptable salt or pharmaceutically acceptable prodrugs, or a stereoisomer thereof and a cytokine as outlined above.

The composition comprising a cytokine and a compound of formula (Ih) in free form or in the form of pharmaceutically acceptable salts and pharmaceutically acceptable prodrugs, may comprise a pharmaceutically acceptable diluent or carrier therefore.

The composition according to the invention comprising a cytokine and a compound of the general formula (Ih) their pharmacologically acceptable salts can be administered to animals, preferably to mammals, and in particular to humans, dogs and chickens as therapeutics per se, as mixtures with one another or in the form of pharmaceutical preparations which allow enteral or parenteral use and which as active constituent contain an effective dose of at least one compound of the formula (Ih) or a salt thereof, in addition to customary pharmaceutically innocuous excipients and additives. The composition comprising a cytokine and the compounds of formula (Ih) can also be administered in form of their salts, which are obtainable by reacting the respective compounds with physiologically acceptable acids and bases.

The production of medicaments containing the composition according to the invention and their application can be performed according to well-known pharmaceutical methods.

While the composition according to the invention comprising a cytokine and compounds of formula (Ih), for use in therapy may be administered in the form of the raw chemical compound, it is preferred to introduce the active ingredients, optionally in the form of a physiologically acceptable salts in a pharmaceutical composition together with one or more adjuvants, excipients, carriers, buffers, diluents, and/or other customary pharmaceutical auxiliaries. Such salts of the compounds may be anhydrous or solvated.

The carrier(s) must be "acceptable" in the sense of being compatible with the other ingredients of the formulation and not harmful to the recipient thereof.

A medicament of the invention may be those suitable for oral, rectal, bronchial, nasal, topical, buccal, sub-lingual, transdermal, vaginal or parenteral (including cutaneous, subcutaneous, intramuscular, intraperitoneal, intravenous, intraarterial, intracerebral, intraocular injection or infusion) administration, or those in a form suitable for administration by inhalation or insufflation, including powders and liquid aerosol administration, or by sustained release systems. Suitable examples of sustained release systems include semipermeable matrices of solid hydrophobic polymers containing the compound of the invention, which matrices may be in form of shaped articles, e.g. films or microcapsules.

The composition according to the invention, together with a conventional adjuvant, carrier, or diluent, may thus be placed into the form of medicament and unit dosages thereof. Such forms include solids, and in particular tablets, filled capsules, powder and pellet forms, and liquids, in particular aqueous or non-aqueous solutions, suspensions, emulsions, elixirs, and capsules filled with the same, all for oral use, suppositories for rectal administration, and sterile injectable solutions for parenteral use. Such Medicament and unit dosage forms thereof may comprise conventional ingredients in conventional proportions, with or without additional active compounds or principles, and such unit dosage forms may contain any suitable effective amount of the active ingredient commensurate with the intended daily dosage range to be employed.

The composition useable according to the invention can be administered in a wide variety of oral and parenteral dosage forms.

For preparing a medicament from a composition according to the invention the coposition comprising a cytokine and compounds of formula (Ih) or pharmaceutically acceptable carriers can be either solid or liquid. Solid form preparations include powders, tablets, pills, capsules, cachets, suppositories, and dispersible granules. A solid carrier can be one or more substances which may also act as diluents, flavouring agents, solubilizers, lubricants, suspending agents, binders, preservatives, tablet disintegrating agents, or an encapsulating material.

In powders, the carrier is a finely divided solid which is in a mixture with the finely divided active component. In tablets, the active component is mixed with the carrier having the necessary binding capacity in suitable proportions and compacted in the shape and size desired. Suitable carriers are magnesium carbonate, magnesium stearate, talc, sugar, lactose, pectin, dextrin, starch, gelatin, tragacanth, methylcellulose, sodium carboxymethylcellulose, a low melting wax, cocoa butter, and the like. The term "preparation" is intended to include the formulation of the active compound with encapsulating material as carrier providing a capsule in which the active component, with or without carriers, is surrounded by a carrier, which is thus in association with it. Similarly, cachets and lozenges are included. Tablets, powders, capsules, pills, cachets, and lozenges can be used as solid forms suitable for oral administration.

For preparing suppositories, a low melting wax, such as a mixture of fatty acid glyceride or cocoa butter, is first melted and the active component is dispersed homogeneously therein, as by stirring. The molten homogenous mixture is then poured into convenient sized moulds, allowed to cool, and thereby to solidify. Compositions suitable for vaginal administration may be presented as pessaries, tampons, creams, gels, pastes, foams or sprays containing in addition to the active ingredient such carriers as are known in the art to be appropriate. Liquid preparations include solutions, suspensions, and emulsions, for example, water or water-propylene glycol solutions. For example, parenteral injection liquid preparations can be formulated as solutions in aqueous polyethylene glycol solution.

The composition comprising a cytokine and compounds of formula (Ih) according to the present invention may thus be formulated for parenteral administration (e.g. by injection, for example bolus injection or continuous infusion) and may be presented in unit dose form in ampoules, pre-filled syringes, small volume infusion or in multi-dose containers with an added preservative. The compositions may take such forms as suspensions, solutions, or emulsions in oily or aqueous vehicles, and may contain formulation agents such as suspending, stabilising and/or dispersing agents. Alternatively, the active ingredient may be in powder form, obtained by aseptic isolation of sterile solid or by lyophilization from solution, for constitution with a suitable vehicle, e.g. sterile, pyrogen-free water, before use.

Aqueous solutions suitable for oral use can be prepared by dissolving the active component in water and adding suitable colorants, flavours, stabilising and thickening agents, as desired. Aqueous suspensions suitable for oral use can be made by dispersing the finely divided active component in water with viscous material, such as natural or synthetic gums, resins, methylcellulose, sodium carboxymethylcellulose, or other well known suspending agents.

Also included are solid form preparations which are intended to be converted, shortly before use, to liquid form preparations for oral administration. Such liquid forms include solutions, suspensions, and emulsions. These preparations may contain, in addition to the active component, colorants, flavours, stabilisers, buffers, artificial and natural sweeteners, dispersants, thickeners, solubilizing agents, and the like.

In one embodiment of the present invention, the medicament is applied topically or systemically or via a combination of the two routes.

Preferably the medicament is prepared in form of an ointment, a gel, a plaster, an emulsion, a lotion, a foam, a cream of a mixed phase or amphiphilic emulsion system (oil/water-water/oil mixed phase), a liposome, a transfersome, a paste or a powder.

Ointments and creams may, for example, be formulated with an aqueous or oily base with the addition of suitable thickening and/or gelling agents. Lotions may be formulated with an aqueous or oily base and will in general also contain one or more emulsifying agents, stabilising agents, dispersing agents, suspending agents, thickening agents, or colouring agents.

Compositions suitable for topical administration in the mouth include lozenges comprising the active agent in a flavoured base, usually sucrose and acacia or tragacanth; pastilles comprising the active ingredient in an inert base such as gelatin and glycerine or sucrose and acacia; and mouthwashes comprising the active ingredient in a suitable liquid carrier.

Solutions or suspensions are applied directly to the nasal cavity by conventional means, for example with a dropper, pipette or spray. The compositions may be provided in single or multi-dose form. In the latter case of a dropper or pipette, this may be achieved by the patient administering an appropriate, predetermined volume of the solution or suspension. In the case of a spray, this may be achieved for example by means of a metering atomising spray pump.

Administration to the respiratory tract may also be achieved by means of an aerosol formulation in which the active ingredient is provided in a pressurised pack with a suitable propellant such as a chlorofluorocarbon (CFC) for example dichlorodifluoromethane, trichlorofluoromethane, or dichlorotetrafluoroethane, carbon dioxide, or other suitable gas. The aerosol may conveniently also contain a surfactant such as lecithin. The dose of drug may be controlled by provision of a metered valve.

Alternatively the active ingredients may be provided in the form of a dry powder, for example a powder mix of the compound in a suitable powder base such as lactose, starch, starch derivatives such as hydroxypropylmethyl cellulose and polyvinylpyrrolidone (PVP). Conveniently the powder carrier will form a gel in the nasal cavity The powder composition may be presented in unit dose form for example in capsules or cartridges of, e.g., gelatin, or blister packs from which the powder may be administered by means of an inhaler.

In compositions intended for administration to the respiratory tract, including intranasal compositions, the compound will generally have a small particle size for example of the order of 5 microns or less. Such a particle size may be obtained by means known in the art, for example by micronization.

When desired, compositions adapted to give sustained release of the active ingredient may be employed.

The pharmaceutical preparations are preferably in unit dosage forms. In such form, the preparation is subdivided into unit doses containing appropriate quantities of the active component. The unit dosage form can be a packaged preparation, the package containing discrete quantities of preparation, such as packaged tablets, capsules, and powders in vials or ampoules. Also, the unit dosage form can be a capsule, tablet, cachet, or lozenge itself, or it can be the appropriate number of any of these in packaged form. Tablets or capsules for oral administration and liquids for intravenous administration and continuous infusion are preferred compositions.

In a preferred embodiment, the composition is formulated in accordance with routine procedures as a pharmaceutical composition adapted for intravenous administration to human beings. Typically, compositions for intravenous administration are solutions in sterile isotonic aqueous buffer. Where necessary, the composition may also include a solubilizing agent and a local anesthetic such as lidocaine to ease pain at the site of the injection. Generally, the ingredients are supplied either separately or mixed together in unit dosage form, for example, as a dry lyophilized powder or water-free concentrate in a hermetically-sealed container such as an ampule or sachette indicating the quantity of active agent. Where the composition is to be administered by infusion, it can be dispensed with an infusion bottle containing sterile pharmaceutical grade water or saline. Where the composition is administered by injection, an ampule of sterile saline can be provided so that the ingredients may be mixed prior to administration.

The invention also provides a pharmaceutical pack or kit comprising one or more containers filled with one or more of the ingredients of the pharmaceutical compositions of the invention. Optionally associated with such container(s) can be a notice in the form prescribed by a governmental agency regulating the manufacture, use or sale of pharmaceuticals or biological products, which notice reflects approval by the agency of manufacture, use or sale for human administration. Thus, the invention also relates to a pack or kit comprising a cytokine and separately therein a compound according to the general formula (1h).

In another embodiment, for example, the cytokine in the composition can be delivered in a controlled-release system. For example, the polypeptide may be administered using intravenous infusion, an implantable osmotic pump, a transdermal patch, liposomes, or other modes of administration. In one embodiment, a pump may be used (see Sefton (1987) CRC Crit. Ref. Biomed. Eng. 14: 201; Buchwald et al. (1980) Surgery 88:507; Saudek et al. (1989) N. Eng, J. Med. 321: 574). In another embodiment, the compound (Ih) can be delivered in a vesicle, in particular a liposome (see Langer (1990) Science 249:1527-1533; Treat et al. (1989) in Liposomes in the Therapy of Infectious Disease and Cancer, Lopez-Berestein and Fidler (eds.), Liss, N.Y., 353-365; WO 91/04014; U.S. 4,704,355). In another embodiment, polymeric materials can be used (see Medical Applications of Controlled Release (1974) Langer and Wise (eds.), CRC Press: Boca Raton, Fla.; Controlled Drug Bioavailability, Drug Product Design and Performance, (1984) Smolen and Ball (eds.), Wiley: N.Y.; Ranger and Peppas (1953) J. Macromol. Sci. Rev. Macromol. Chem. 23: 61; see also Levy et al. (1985) Science 228:190; During et al. (1989) Ann. Neurol. 25: 351; Howard et al. (1989) J. Neurosurg. 71: 105).

In yet another embodiment, a controlled release system can be placed in proximity of the therapeutic target, i.e., the target cells, tissue or organ, thus requiring only a fraction of the systemic dose (see, e.g., Goodson (1984) 115-138 in Medical Applications of Controlled Release, vol. 2). Other controlled release systems are discussed in the review by Langer (1990, Science 249: 1527-1533).

In a specific embodiment, it may be desirable to administer the pharmaceutical compositions of the invention locally to the area in need of treatment; this may be achieved by, for example, and not by way of limitation, local infusion during surgery, topical application, e.g., in conjunction with a wound dressing after surgery, by injection, by means of a catheter, by means of a suppository, or by means of an implant, said implant being of a porous, nonporous, or gelatinous material, including membranes, such as silastic membranes, or fibers.

Further details on techniques for formulation and administration may be found in the latest edition of Remington's Pharmaceutical Sciences (Maack Publishing Co. Easton, Pa.).

Pharmaceutical compositions can also contain two or more compounds of the formula (Ih) or their pharmacologically acceptable salts and also other therapeutically active substances. Pharmaceutical compositions can also contain two or more cytokines and also other therapeutically active substances.

Suitable amounts of compound (Ih) to be administered to humans may range from 5 to 500 mg.

To prepare the pharmaceutical preparations, pharmaceutically inert inorganic or organic excipients can be used. To prepare pills, tablets, coated tablets and hard gelatin capsules, for example, lactose, corn starch or derivatives thereof, talc, stearic acid or its salts, etc. can be used. Excipients for soft gelatin capsules and suppositories are, for example, fats, waxes, semi-solid and liquid polyols, natural or hardened oils etc. Suitable excipients for the production of solutions and syrups are, for example, water, sucrose, invert sugar, glucose, polyols etc. Suitable excipients for the production of injection solutions are, for example, water, alcohols, glycerol, polyols or vegetable oils.

The dose of formula (Ih) in the composition can vary within wide limits and is to be suited to the individual conditions in each individual case. For the above uses the appropriate dosage will vary depending on the mode of administration, the particular condition to be treated and the effect desired. In general, however, satisfactory results are achieved at dosage rates of about 1 to 100 mg/kg animal body weight preferably 1 to 50 mg/kg. In general, suitable dosage rates for larger mammals, for example humans, may be of the order of from about 10 mg to 3 g/day, conveniently administered once, in divided doses 2 to 4 times a day, or in sustained release form.

With respect to compound (Ih) in the composition, a daily dose of approximately 10 mg to 5000 mg, preferably 50 to 500 mg, per human individual is appropriate in the case of the oral administration. In the case of other administration forms too, the daily dose is in similar ranges. For topical delivery, depending on the permeability of the skin, the type and the severity of the disease and dependent on the type of formulation and frequency of application, different concentrations of active compounds within the medicament can be sufficient to elicit a therapeutic effect by topical application. Preferably the concentration of an active compound or a pharmaceutically acceptable salt thereof or a physiologically functional derivative or a stereoisomer thereof within a medicament according to the invention is in the range of between 1 µmol/l and 100 mmol/l.

The following examples and figures are included to demonstrate preferred embodiments of the invention. It should be appreciated by those of skill in the art that the techniques disclosed in the examples that follow represent techniques discovered by the inventors to function well in the practice of the invention, and thus can be considered preferred modes for its practice.

However, those of skill in the art should, in light of the present disclosure, appreciate that many changes can be made in the specific embodiments that are disclosed without departing from the spirit and scope of the invention as set out in the appended claims. All references cited are incorporated herein by reference.

### Examples

Abbreviations: min, minute(s); h, hour(s); r.t., room temperature; t-, tert-. NMR spectra: Bruker Avance 300 MHz. The spectra were recorded at 300 MHz (¹H-NMR), respectively, using the residual solvent peak as an internal standard (DMSO-d₆, δ_{H} = 2.49; CD₃OD, δ_{H} = 3.31; CDCl₃, δ_{H} = 7.26; CD₃CN, δ_{H} = 1.93; (CD₃)₂CO, δ_{H} = 2.05).
Analytical LC/ESI-MS: 2 x Waters 600 Multisolvent Delivery System. 50 µl sample loop. Column, Chromolith Speed ROD RP18e (Merck, Darmstadt), 50 x 4.6 mm, with 2 µm prefilter (Merck). Eluent A, H₂O + 0.1 % HCO₂H; eluent B, MeCN. Gradient, 5 % B to 100 % B within 5 min; flow, 3 ml/min. Waters LCZ single quadrupol mass spectrometer with electrospray source. MS method, MS8minPM-80-800-20V; positive/negative ion mode scanning, m/z 80 - 800 in 1 s; capillary, 3.5 kV; cone voltage, 20 V; multiplier voltage, 400 V; probe and desolvation gas temperature, 120° C and 350° C, respectively. Waters 2487 Dual λ Absorbance Detector, set to 254 nm.

Preparative HPLC-MS: Waters 600 Multisolvent Delivery System with peparative pump heads. 2000 µl or 5000 µl sample loop. Column, Waters X-Terra RP18, 7 µm, 19 x 150 mm with X-Terra RP18 guard cartridge 7 µm, 19 x 10 mm; used at flow rate 20 ml/min or YMC ODS-A, 120 Å, 40 x 150 mm with X-Terra RP18 guard cartridge 7 µm, 19 x 10 mm; used at flow rate 50 ml/min. Make-up solvent: MeCN - H₂O - HCO₂H 80 : 20 : 0.05 (v:v:v). Eluent A, H₂O + 0.1 % HCO₂H; eluent B, MeCN. Different linear gradients from 5 - 100% eluent B, adapted to sample. Injection volume: 500 µl - 2000 µl depending on sample. Waters ZQ single quadrupol mass spectrometer with electrospray source. Positive or negative ion mode scanning *m*/*z* 80 - 800 in 1 s; capillary, 3.5 kV or 3.0 kV; cone voltage, 20 V; multiplier voltage, 400 V; probe and desolvation gas temperature, 120° C and 350° C, respectively. Waters Fraction Collector II with mass-triggered fraction collection. Waters 996 photo diode array detector.

### Synthesis of (Ih)

The compounds of formula (Ih) may be obtained *via* various methods.

Piperidin-4yl-thiazole-4-carboxamide can be prepared by various methodes described in the literature. One such example is the oxidation of the appropriate 2,5-dihydrothiazoles as described in Houben-Weyl, 2002, 730. The dihydrothiazoles can also be synthesised by methodes described in the same reference or described in You, S., Razavi, H., Kelly, J.W. Angew. Chem. 2003, 115, 87 or Katritzky, AR., Cai, C., Suzuki, K., Singh, SK. J. Org. Chem. 2004, 69, 81I-814 and references in both papers. Alternative methods were described by Yasuchika, S. et. al. Heterocycles, Vol. 57, No. 5, 2002**.**

Compounds of the present invention carrying a piperidin-4-yl substituent in the 2-position of the thiazole ring can, for example, be prepared as shown in the following scheme. This synthetic route is partially described in WO 2004/058750.

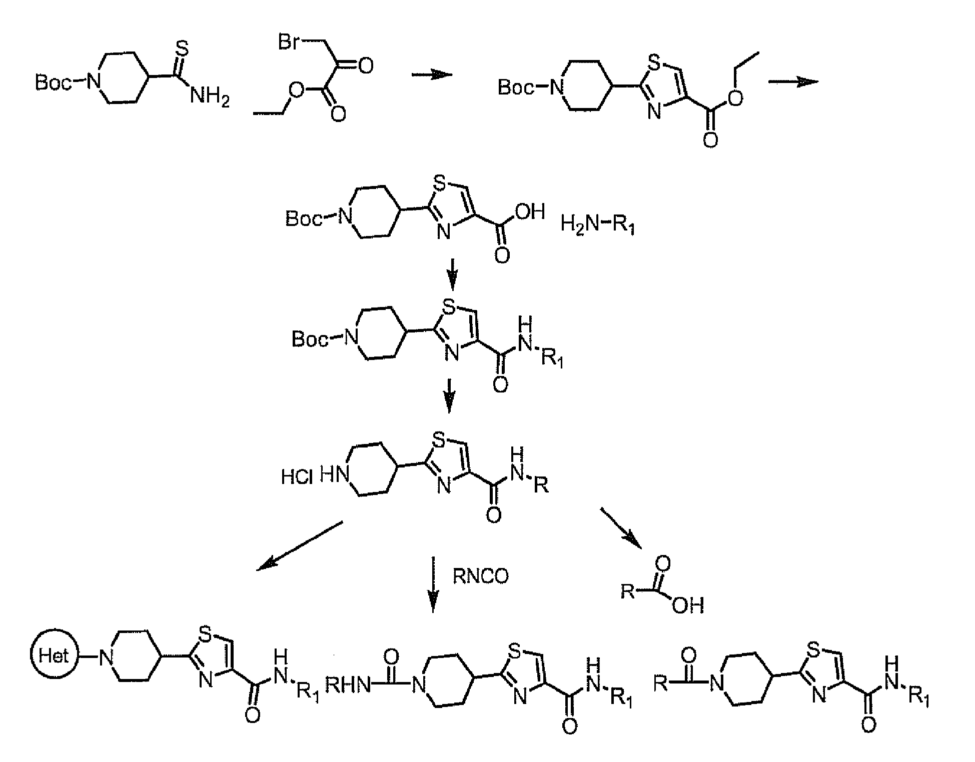

2-(1-(tert-butoxycarbonyl)piperidin-4-yl)thiazole-4-carboxylic acid can be converted to the appropriate R¹ amide by coupling with HBTU, DIPEA in DMF. The different R¹-amines are either commercially available or can be readily synthesized. The Boc-protection group can be removed under standard conditions, such as treatment with TFA for 2 to 3 hours at 0° or with 4 N HCl in dioxane for 2 to 3 hours. The deprotected piperidinoderivative HCl salt can then be converted to the corresponding amides, ureas and N-heterocyclic analogs as follows.

Urea substituted piperidino compounds can be synthesized by coupling with commercially available isocyanates in the presence of DIPEA. Piperidino compounds substituted with heterocycles can be synthesized by standard procedures, such as coupling with the corresponding chloroheterocycle in the presence of a base. Alternatively, piperidino compounds substituted with heterocycles can be obtained by palladium-mediated cross coupling. As a further alternative, hydroxypyridine derivatives can be coupled to the piperidino compound by the HBTU coupling method.

An alternative route to compounds of the present invention carrying a piperidin-4-yl substituent in the 2-position of the thiazole ring is shown in the following scheme.

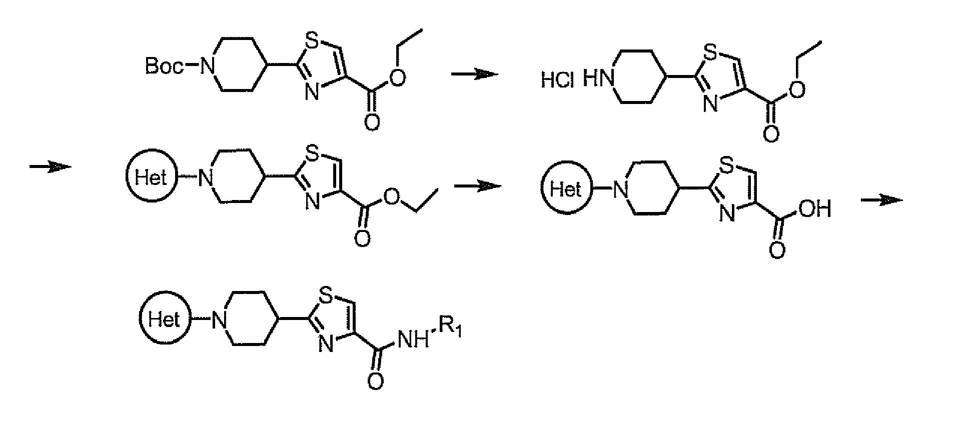

The Boc-protection group can be removed under standard conditions, such as treatment with TFA for 2 to 3 hours at 0° or with 4 N HCl in dioxane for 2 to 3 hours. The deprotected piperidinoderivative HCl salt can then be converted to the corresponding N-heterocyclic analog by various methods as described as described above.

Synthesis of 4-(Methoxy-methyl-carbamoyl)-piperidine-1-carboxylic acid t-butyl ester Piperidine-1,4-dicarboxylic acid mono-tert-butyl ester (1.0 eq, 21.8 mmol) was dissolved under inert conditions in 35 ml dry N, N- dimethylformamide, O,N-dimethyl-hydroxylamine hydrochloride (1.03 eq, 22.5 mmol), benzotriazol-1-ol monohydrate (1.03 eq, 22.5 mmol) and triethylamine (1.5 eq, 32.7 mmol) were added. The reaction mixture was cooled to 0°C, N-(3-Dimethylaminopropyl)-N-ethylcarbodiimid hydrochloride (1.0 eq, 21.8 mmol) was added over a period of 10 minutes and the mixture was stirred vigorously at 0°C for 1h and at r.t. for 18h.

The solvent was removed under vaccum and the residue was suspended in 400 ml ethylacetate. The organic layer was extracted 3 times with 100 ml of 1 M citric acid, aqueous sodium carbonate and twice with 100 ml brine, dried over MgSO₄ and filtered. The solvent was removed and the residue was purified by distillation resulting in a yield of 80%.

### Synthesis of 4-Formyl-piperidine-1-carboxylic acid t-butyl ester

4-(methoxy-methyl-carbanloyl)-piperidine-1-carboxylic acid tert-butyl ester (1.0 eq, 16.4 mmol) was dissolved in 100 ml dry tetrahydrofurane under inert atmosphere. This solution was added dropwise over a period of 1 h to a suspension of lithiumalanate (3.0 eq, 49.6 mmol) in 70 ml dry tetrahydrofurane at -50°C. During the adding of the mixture, the temperature was held at - 50°C and then allowed to warm to 0°C within 3 h.

The mixture was cooled to -78 °C and quenched carefully with 100 ml 1 M citric acid. The mixture was warmed up to r.t. and diluted with 400 ml ethylacetate. The phases were separated and the aqueous phase was extracted 3 times with 70 ml ethylacetate. The combined organic layers were extracted 3 times with 100 ml 1 M citric acid, aqueous sodium carbonate and 2 times with 100 ml brine, dried over MgSO₄ and filtrated. The solvent was removed and the residue was purified by distillation resulting in a yield of 85%

### Synthesis of 4-(4-Ethoxycarbonyl-4,5-dihydro-thiazol-2-yl)-piperidine-1-carboxylic acid t-butyl ester

4-formyl-piperidine-1-carboxylic acid tert-butyl ester (1.0 eq, 13 mmol) was dissolved under inert conditions in 40 ml toluene. To this solution L-cystein ethylester hydrochloride (1.6 eq, 21 mmol) and triethylamine (1.6 eq, 21 mmol) were added. The mixture was refluxed for 14 h. The generated water was removed with a Dean & Stark trap.

The solvent was removed and the residue was dissolved in 100 ml ethylacetate. The organic layer was extracted 3 times with 50 ml 1 M citric acid, aqueous potassium hydrogen carbonate and 2 times with 50 ml brine, dried over MgSO₄ and filtrated. The solvent was removed and the residue was purified by silica gel chromatography using a PE/ EA 4:1 gradient. Yield: 75%

### Synthesis of 4-(4-Ethoxycarbonyl-thiazol-2-yl)-piperidine-I -carboxylic acid t-butyl ester

4-(4-Ethoxycarbonyl-4,5-dihydro-thiazol-2-yl)-piperidine-1-carboxylic acid tert-butyl ester (1.0 eq, 8.7 mmol) was solved in 160 ml toluene under inert conditions. To this solution MnO₂ (15.0 eq, 130 mmol) was added. The reaction was heated to 70 °C under stirring for 5 h. The mixture was filtered over celite and the filtration agent was washed 3 times with 30 ml toluene and ethylacetate. The combined organic layers were distilled in vacuo. The residue was purified by silica gel chromatography using a DCM/MeOH 95:5 gradient. Yield: 30%

### C-terminal functionalisation

4-(4-Ethoxycarbonyl-thiazol-2-yl)-piperidine-1-carboxylic acid tert-butyl ester (1.0 eq, 2.9 mmol) was dissolved under inert gas in 40 ml dioxane. Under stirring 1.5 ml aqueous 2 N NaOH was added dropwise over a period of 10 min. Afterwards the mixture was stirred for 2 h at r.t.

The reaction was neutralized with 2 N HCl and the solvent was evaporated in vacuo. The residue was dissolved in 50 ml ethylacetate. The organic layer was extracted 3 times with 10 ml of 1 M citric acid and water, dried over MgSO₄ and filtered. The solvent was removed and the residue was dried in vacuo. Yield 95%
4-(4-Carboxy-thiazol-2-yl)-piperidine-1-carboxylic acid tert-butyl ester (1 eq) was dissolved under inert conditions in dry dimethylacetamide (0,03 mmol/ml). To this solution aryl- or alkylamine (1 eq), diisopropylethylamine (2 eq) and O-benzotriazol-yl-N,N,N',N'-tetramethyluronium hexafluorophosphate (2 eq) was added. The reaction mixture was stirred for 12 h at r.t.

The solvent was removed in vacuo and the residue was dissolved in ethylacetate. The organic layer was extracted 3 times with 1 M citric acid, aqueous potassium hydrogen carbonate and 2 times with brine, dried over MgSO₄ and filtred. The solvent was removed and the residue was purified by silica gel chromatography using a DCM/MeOH 95:5gradient. Yield: 40-80%

### N-terminal functionalisation

The N-protected substrate was treated under inert condition with 4 M HCl/dioxane (conc. 0,03 mmol substrate in 1 mL HCl/dioxane) and was stirred for 2 h at r.t.

The solvent was removed in vacuo to yield the HCl salt of the free amine without further purification.

The free amino compound (1eq) was dissolved under inert conditions in dry dimethylacetamide (0,03 mmol/ml). To this solution aryl- or alkylcarboxylic acid (1 eq), diisopropylethylamine (2 eq) and O-benzotriazol-1-yl-N,N,N',N'-tetramethyluronium hexafluorophosphate (2 eq) was added in this sequence and the reaction mixture was stirred for 12 h at r.t.

The solvent was removed in vacuo and the residue was dissolved in ethylacetate. The organic layer was extracted 3 times with 1 M citric acid, aqueous potassium hydrogen carbonate and 2 times with brine, dried over MgSO₄ and filtred. The solvent was removed and the residue was purified by silica gel chromatography using a DCM/MeOH 95:5 gradient. Yield: 40-80%

Exemplary compounds of formula (Ih) of the present invention include, but are not limited to, the following:

| Cp. | **Name** | Mass | |
|---|---|---|---|
| 273 | 2-(1-Thieno[3,2-d]pyrimidin-4-yl-piperidin-4- yl)-thiazole-4-carboxylic acid (5-b enzoyl-1H-benzaimidazol-2-yl)-amide | 565 | |
| 279 | 2-(1-(2,6-dimethoxypyrimidin-4-yl)piperidin-4-yl)-N-(6-(thiophene-3-carbonyl)-1H-benzo[d]imidazol-2-yl)thiazole-4-carboxamide | 575 | |
| 280 | 2-(1-(thieno [3,2-d] pyrimidin-4-yl)piperidin-4-yl)-N-(6-(thiophene-3-carbonyl)-1H-benzo [d]imidazol-2-yl)thiazole-4-carboxamide | 571 | |
| 281 | 2-(1-(4,6-dimethoxy-1,3,5-triazin-2-yl)piperidin-4-yl)-N-(6-(thiophene-3-carbonyl)-1H -benzo[d]imidazol-2-yl)thiazole-4-carboxamide | 576 | |
| 282 | N-(6-benzoyl-I H-benzo[d]imidazol-2-yl)-2-(1-(2,6-dimethoxypyrimidin-4-yl)piperidin-4-yl)thiazole-4-carboxamide | 569 | |
| 283 | N-(5-acetylbenzo[d]tlliazol-2-yl)-2-(1-(thieno[3,2-d]pyrimidin-4-yl)piperidin-4-yl)thiazole-4-carboxamide | 520 | |
| 284 | N-(5-(3-fluoro-4-methoxybenzoyl)benzo[d]thiazol-2-yl)-2-(1-(thieno[3,2-d]pyrimidin-4-yl)piperidin-4-yl)thiazole-4-carboxamide | 630 | |
| 285 | N-(5-(4-hydroxy-3-methoxybenzoyl)benzo[d]thiaxol-2-yl)-2-(1-(thieno[3,2-d]pyrimidin-4-yl)piperidin-4-yl)thiazole-4-carboxamide | 628 | |
| 286 | N-(6-benzoyl-1H-benzo[d]imidazol-2-yl)-2-(1-(thiophen-2-ylsulfonyl)piperidin-4-yl)thiazole-4-carboxamide | 577 | |
| 287 | N-(5-benzoyl-1 H-benzo[d]imidazol-2-yl)-2-(1-(6,7-dimethoxyquinazolin-4-yl)piperidin-4-yl)thiazole-4-carboxamide | 619 | |
| 288 | N-(5-(3-methoxybenzoyl)benzo[d]thiazol-2-yl)-2-(1-(thieno[3,2-d]pyrimidin-4-yl)piperidin-4-yl)thiazole-4-carboxamide | 612 | |
| 289 | 2-(1-(7H-purin-6-yl)piperidin-4-yl)-N-(6-benzoyl-1H-benzo[d]imidazol-2-yl)thiazole-4-carboxamide | 549 | |
| 290 | N-(6-benzoyl-1H-benzo[d]imidazol-2-yl)-2-(1-(2,6-dichloropyridin-4-ylcarbamoyl)piperidin-4-yl)thiazole-4-carboxamide | 619 | |
| 291 | N-(6-benzoyl-1H-benza[d]imidazol-2-yl)-2-(1-(4-cyanopyridin-2-yl)piperidin-4-yl)thiazole-4-carboxamide | 533 | |
| 292 | N-(5-benzoyl-1H-benzo[d]imidazol-2-yl)-2-(1-(4,6-dimethoxy-1,3,5-triazin-2-yl)piperidin-4-yl)thiazole-4-carboxamide | 570 | |
| 294 | N-(6-benzoyl-1H-benzo[d]imidazol-2-yl)-2-(1-(6-(trifluoromethyl)pyrimidin-4-yl)piperidin-4-yl)thiazole-4-carboxamide | 577 | |
| 295 | N-(5-benzoyl-1H-benzo[d]imidazol-2-yl)-2-(1-(4-cyanobenzyl)piperidin-4-yl)thiazole-4-carboxamide | 546 | |
| 296 | 2-(1-([1,2,4]triazolo[1,5-a]pyrimidin-7-yl)piperidin-4-yl)-N-(5-benzoyl-1H-benzo[d]imidazal-2-yl)thiazole-4-carboxamide | 549 | |
| 297 | N-(5-benzoyl-I H-benzo[d]imidazol-2-yl)-2-(1-(2-methylpyrazolo[1,5-a]pyrimidin-7-yl)piperidin-4-yl)thiazole-4-carboxamide | 562 | |
| 298 | 4-{4-[4-(5-Benzoyl-1H-benzomidazol-2-ylcarbamoyl)-thiazol-2-yl]-piperidin-1-yl}-5-methyl-pyrrolo[2,1-f][1,2,4]triazine-6-carboxylic acid methyl ester | 620 | |
| 299 | methyl 7-(4-(4-5-benzoyl-1H-benzo[d]imidazal-2-ylcarbamoyl)thiazol-2-yl)piperidin-1-yl)-[1,2,4]triazolo[1,5-a]pyrimidine-2-carhoxylate | 607 | |
| | | | |
| 1 | 2-(1-(2, 6-dimethoxypyrimidin-4-yl)piperidin-4-yl)-N-(6-(thiophene-3-carbonyl)-1H-benzo[d]imidazol-2-yl)thiazole-4-carboxamide | 575 | 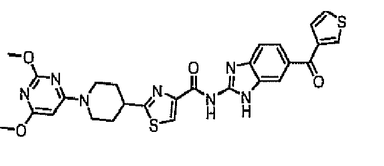 |
| 2 | 2-(1-(thieno[3,2-d]pyrimidin-4-yl)piperidin-4-yl)-N-(6-(thiophene-3-carbonyl)-1H-benzo[d]imidazol-2-yl)thiazole-4-carboxamide | 571 | 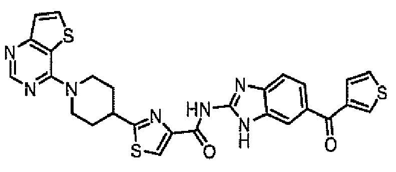 |
| 3 | 2-(1-(4,6-dimethoxy-1,3,5-triazin-2-yl)piperidin-4-yl)-N-(6-(thiophene-3-carbonyl)-1H-benzo[d]imidazol-2-yl)thiazole-4-carboxamide | 576 | 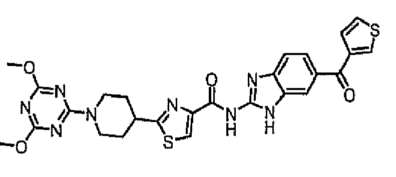 |
| 4 | N-(6-b enzoyl-1H-benzo [d]imidazol-2-yl)-2-(1-(2,6-dimethoxypyrimidin-4-yl)piperidin-4-yl)thiazole-4-carboxamide | 569 | 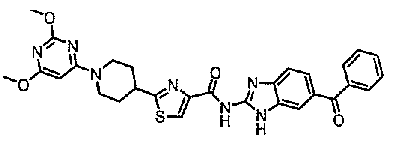 |
| 5 | N-(5-acetylbenzo[d]thiazol-2-yl)-2-(1-(thieno[3,2-d]pyrimidin-4-yl)piperidin-4-yl)thiazole-4-carboxamide | 520 | 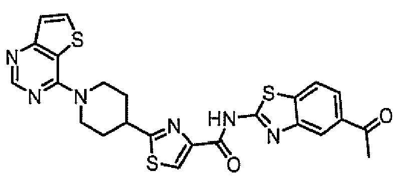 |
| 6 | N-(5-(3-fluoro-4-methoxybenzoyl)benzo[d]thiazol-2-yl)-2-(1-(thieno[3,2-d]pyrimidin-4-yl)piperidin-4-yl)thiazole-4-carboxamide | 630 | 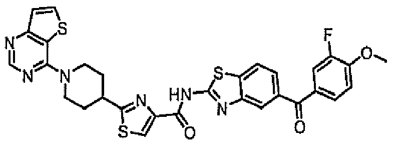 |
| 7 | N-(5-(4-hydroxy-3-methoxybenzoyl)benzo[d]thiazol-2-yl)-2-(1-(thieno [3,2-d]pyrimidin-4-yl)piperidin-4-yl)thiazole-4-carboxamide | 628 | 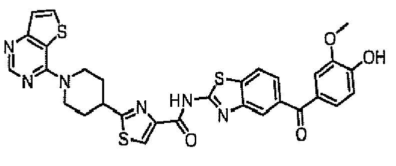 |
| 9 | N-(6-benzoyl-1H-benzo[d]imidazol-2-yl)-2-(1-(thiophen-2-ylsulfonyl)piperidin-4-yl)thiazole-4-carboxamide | 577 | 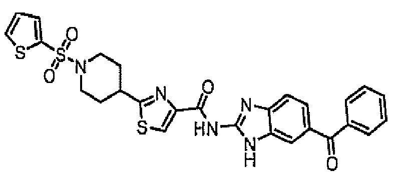 |
| 10 | N-(5-benzoyl-1H-benzo[d]imidazol-2-yl)-2-(1-(6,7-dimethoxyquinazolin-4-yl)piperidin-4-yl)thiazole-4-carboxamide | 619 | 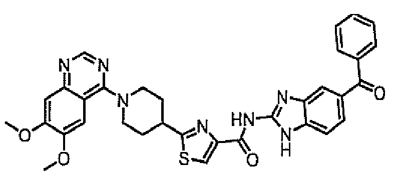 |
| 12 | N-(5-(3-methoxybenzoyl)benzo[d]thiazol-2-yl)-2-(1-(thieno[3,2-d]pyrimidin-4-yl)piperidin-4-yl)thi azole-4- carboxamide | 612 | 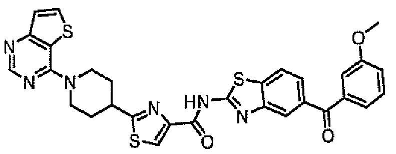 |
| 13 | 2-(1-(7H-purin-6-yl)piperidin-4-yl)-N-(6-benzoyl-1H-benzo[d]imidazol-2-yl)thiazole-4-carboxamide | 549 | 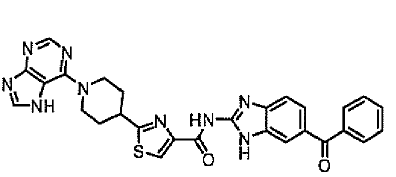 |
| 14 | N-(6-benzoyl-1H-benzo[d] imidazol-2-yl)-2-(1-(2,6-dichloropyridin-4-ylcarbamoyl)piparidin-4-yl)thiazole-4-carboxamide | 619 | 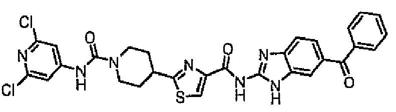 |
| 17 | N-(6-benzoyl-1H-benzo[d]imidazol-2-yl)-2-(1-(4-cyanopyridin-2-yl)piperidin-4-yl)thiazole-4-carboxamide | 533 | 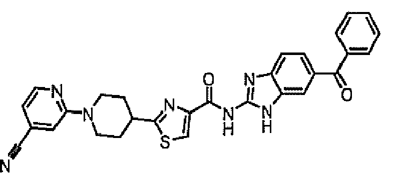 |
| 18 | N-(5-benzoyl-1H-benzo[d]imidazol-2-yl)-2-(1-(4,6-dimethoxy-1,3,5-triazin-2-yl)piperidin-4-yl)thiazole-4-carboxamide | 570 | 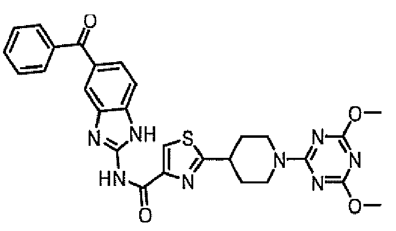 |
| 19 | N-(6-benzoyl-1H-benzo[d]imidazol-2-yl)-2-(1-(thieno[3,2-d]pyrimidin-4-yl)piperidin-4-yl)thiazole-4-carboxamide | 566 | 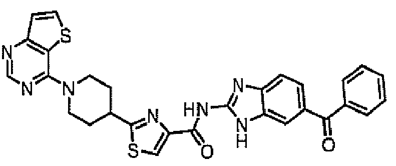 |
| 20 | N-(6-benzoyl-1H-benzo[d]imidazol-2-yl)-2-(1-(6-(trifluoromethyl)pyrimidin-4-yl)piperidin-4-yl)thiazole-4-carboxamide | 577 | 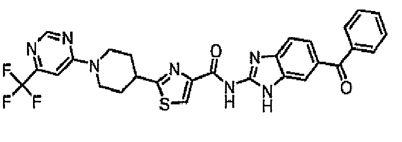 |
| 21 | N-(5-benzoyl-1H-benzo[d]imidazol-2-yl)-2-(1-(4-cyanobenzyl)piperidin-4-yl)thiazole-4-carboxamide | 546 | 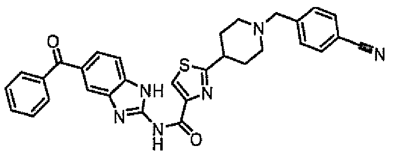 |
| 23 | 2-(1-([1 ,2,4]triazolo[1 ,5-a]pyrimidin-7-yl)piperidin-4-yl)-N-(5-benzoyl-1H-benzo[d]imidazol-2-yl)thiazole-4-carboxamide | 549 | 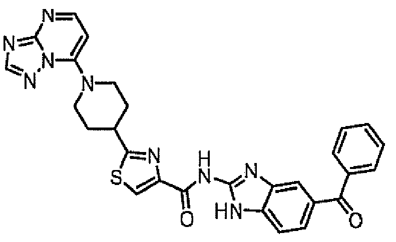 |
| 24 | N-(5-benzoyl-1H-benzo[d]imidazol-2-yl)-2-(-(2-methylpyrazolo[1,5-a]pyrimidin-7-yl)piperidin-4-yl)thiazole-4-carboxamide | 562 | 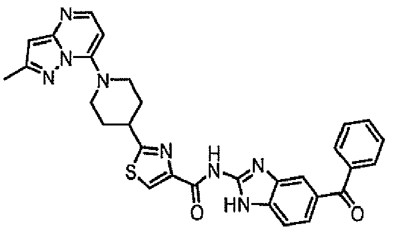 |
| 25 | 4- (4-[4-(5-Benzoyl-1H-berizoimidazol-2-ylcarbamoyl)-thiazol-2-yl]-piperidin-1-yl}-5-methyl-pyrrolo[2,1-f] [1,2,4]triazine-6-carboxylic acid methyl ester | 620 | 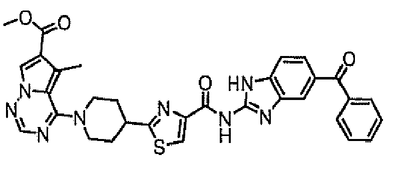 |
| 26 | methyl 7-(4-(4-(5-benzoyl-1H-benzo[d]imidazol-2-ylcarbamayl)thiazol-2-yl)piperidin-1-yl)-[1,2,4]triazolo[1,5-a]pyrimidine-2-carboxylate | 607 | 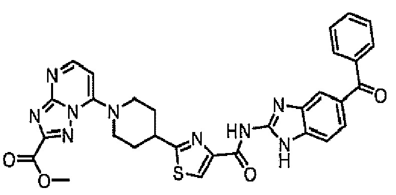 |

### Composition comprising a cytokine and a compound according to general fornula (Ih):

IFN-β, inhibits ConA-induced peripheral blood mononuclear cell (PBMC) proliferation in PBMCs of MS patients and healthy individuals (J Neuroimmunol. 1993; 46(1-2):145-53). We tested the activity of a combination of a substance according to the general formula (Ih) in combination with IFN-β on PBMCs of healthy donors.

Human PBMCs were stimulated with PHA and proliferation inhibition was measured either in the presence of IFN-β or of a substance or formula (Ih). Further, the proliferation inhibition was determined in the presence of both substances.

Further the activity of a combination of IFN-α with a substance of formular (Ih) on a cell line infected with a flavivirus was tested.

### Bovine virus diarrhoe virus (BVDV) assay:

### Drug preparation, i.e. preparation of an exemplary composition according to the invention:

The substances were solubilized in DMSO. Pegylated interferone-alpha (IFN) was supplied by Southern Research Institute and dissolved in sterile dH₂O. Final DMSO concentrations employed in the assays were < 1 % and do not influence the described assays.

### BVDV Efficacy Evaluation:

Drug-drug interactions were assayed by testing both the antiviral and cytotoxic activities of a wide range of concentrations of combinations of the compounds and IFN. The bovine viral diarrhea virus (BVDV) NADL strain antiviral evaluation assay and drug-drug combination analysis was performed as described in Antimicro. Agents Chemother. 2003; 47: 2293-8. BVDV is frequently employed as a hepatitis C virus surrogate model in the study of antiviral agents **(**Antiviral Res. 2003; 60: 1-15**).** Madin-Darby bovine kidney cells were trypsinized, pelleted, counted and resuspended at 1×10⁴/well in tissue culture medium in 96-well flat bottom, tissue culture plates in volume of 100µl per well. One day following plating of cells, the wells were washed and the medium was replaced with complete medium (2% serum) containing various concentrations of test compounds diluted in medium. A pre-titered aliquot of virus was removed from the freezer (-80°C) just before each experiment. The virus was diluted into tissue culture medium such that the amount of virus added to each well would give complete cell killing at 6-7 days post-infection. The format of the test plate for antiviral evaluation has been standardized by Southern Research Institut6e. Each plate contains cell control wells (cells only), virus control wells (cells plus virus), drug cytotoxicity wells (cells plus drug only), drug colorimetric control wells (drug only) as well as experimental wells (drug plus cells plus virus). Drug-drug combination analysis utilizes a checkerboard dilution matrix scheme. Five concentrations of substance were evaluated in combination with nine different concentrations IFN.; antiviral efficacy and cellular toxicity were monitored by MTS staining at the experimental endpoint.

### MTS Staining for cell viability:

At assay termination, the assay plates were stained with the soluble tetrazolium-based dye MTS (CellTiter Reagent Promega) to determine cell viability and quantify compound toxicity. MTS is metabolized y the mitochondrial enzymes of metabolically active cells to yield a soluble formazan product, allowing the rapid quantitative analysis of cell viability and compound cytotoxicity. The MTS is a stable solution that does not require preparation before use. At termination of the assay, 10 µl of MTS reagent was added per well. The microtiter plates were then incubated four hours at 37°C. Adhesive sealers were used in place of the lids, the sealed plate was inverted several times to mix the soluble formazan product and the plate was read spectrophotometrically at 490 nm and 650 nm with a Molecular Devices Vmax plate reader.

### Data analysis:

The drug combination assay data was analyzed according to the method of Prichard and Shipman (Mac Synergy II. Version 1.0 User's manual. 54pp.) useng the MacSynergy II program for data analysis and statistical evaluation. Briefly, the Mac Synergy II program calculates the theoretical additive interactions of the drugs based in the Bliss Independence mathematical definition of expected effects for drug-drug interactions. The Bliss independence model is based on statistical probability and assumes that the drugs act independently to affect virus replication.

Theoretical additive values are calculated from the dose response curves for each substance used individually. This calculated additive surface, which represents predicted or additive interactions, is then subtracted from the experimentally determined dose response surface to reveal regions of non-additive activity. The resulting surface would appear as a horizontal plane at 0% inhibition above calculated it the interactions were merely additive. Any peaks above this plane-of-additivity would be indicative of synergy. Similarly, any depressions below the plane-of-additivity would indicate antagonism. The 95% confidence intervals around the experimental dose-response surface are used to evaluate the data statistically and the volume of the peaks/depressions is calculated and used to quantify the volume of synergy/antagonism produced. The volume of the peaks observed in the synergy plots (in units of concentration times concentration times percent; generally µM²%) was calculated by the program. This peak volume is the three-dimensional counterpart of the area under a three-dimensional dose-response surface and is a quantitative measure of synergy or antagonism. For these studies, minor synergy or antagonism is indicated when synergy or antagonism volumes are between 25-50 µM²%, Moderate effects are indicated by synergy and antagonism volumes of 50-100 µM²%, while strong effects are noted when the synergy or antagonism volumes are > 100 µM²%. Drug synergy or antagonism is indicting by a reproducible clustering of adjacent cells above or below the zero plane in the different plots.

### T-Lymphocyte Proliferation Assay:

Inhibition of stimulated peripheral blood monocytes (PBMC). PBMCs were isolated from the blood of healthy volunteers with the help of ACCUSPIN^{™} System Histopaque^{®}-1077 tubes, washed and resuspended with 10⁶ cells/ml in Dulbecco's modified eagles medium, containing 10 % fetal calf serum and 2 mM Glutamine. The cells were stimulated with 2 µg/ml phytohemoagglutinin in the presence of test compound or blank vehicle for 72 h. 4 h prior to the end of the incubation period, 5'-brarno-2'-desoxyuridine (BrdU) was added to label the proliferating cells. After the incubation, the cells were separated by centrifugation and the culture supernatant removed. Incorporated BrdU was quantified with the help of an enzyme-linked immunosorbent assay (Roche Diagnostics, Mannheim, Germany).

For the determination of the IC₅₀ values (concentration of inhibitor required for 50% inhibition) at least four different inhibitor concentrations were applied. Each data point was recorded in triplicates. Curves were fitted with a suitable program.

### Results:

### BVDV assay:

Compound 273 in combination with TNF-α showed highly synergistic behaviour concerning their combined antiviral activity, whereas concerning their combined cytotoxic activity, they showed highly antagonistic behaviour. Thus, the compounds of formula (Ih) in combination with cytokines, i.e. interferones, are suitable for treating viral diseases, especially diseases caused by flaviviruses.

### PBMC assay:

Compounds according to the examples resulted in an inhibition of more than 50% compared to control experiments. The average EC50 of the compounds were between 3 and 40µM. Coadministration of INF-α resulted at least in an additive effect concerning activity. Thus, the compounds of formula (1h) in combination with cytokines, *i.e.* interferones, are ideal for treating inflammatory diseases or diseases associated with T-cells.

### Figure captions

### Fig. 1

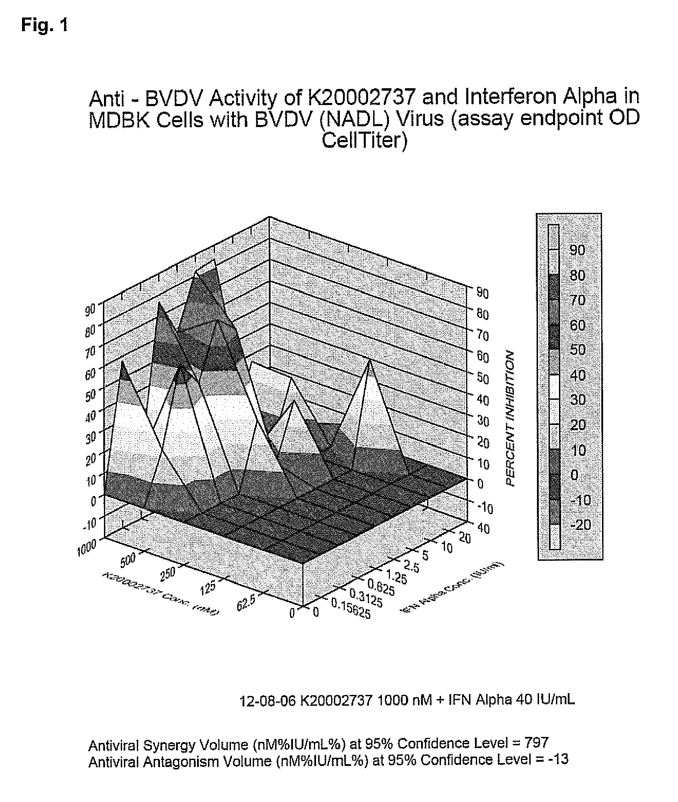

Figure 1 shows anti-BVDV activity of compound 273 (see table herein) and INF-α in MDBK cells with BVDV virus. Further details concerning data evaluation are also outlined above in the passage relating to the BVDV assay - data analysis. In short: additive behaviour of the two compounds would be represented by a horizontal plane, synergistic behaviour by peaks above and antagonistic behaviour by depressions below the plane. The 95% confidence intervals around the experimental dose-response surface are used to evaluate the data statistically and the volume of the peaks/depressions is calculated and used to quantify the volume of synergy/antagonism produced.

### Fig. 2

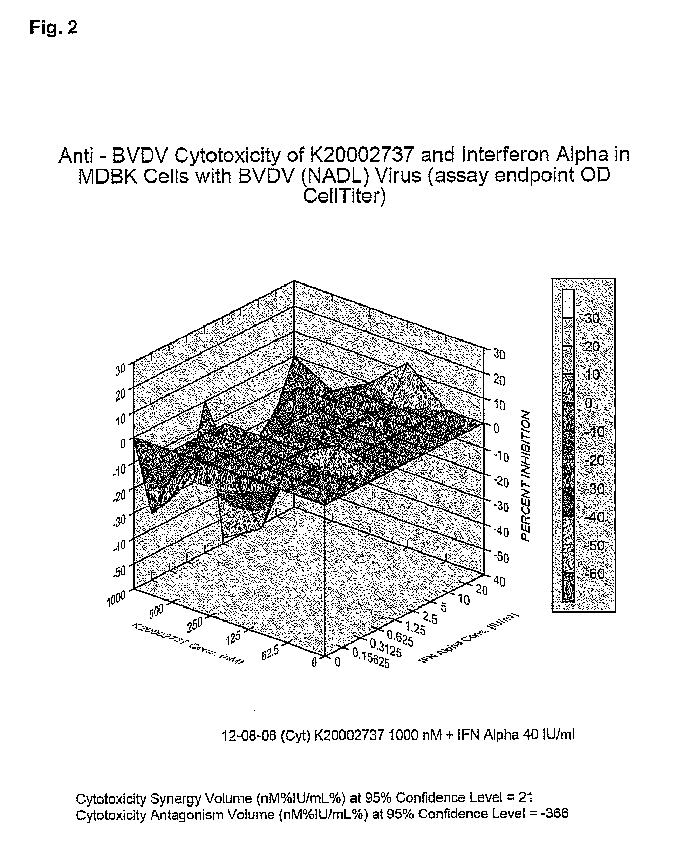

Figure 2 shows anti-BVDV cytotoxicity of compound 273 and INF-α in MDBK cells with BVDV virus. Further details concerning data evaluation are also outlined above in the passage relating to the BVDV assay - data analysis. In short: additive behaviour of the two compounds would be represented by a horizontal plane, synergistic behaviour by peaks above and antagonistic behaviour by depressions below the plane. The 95% confidence intervals around the experimental dose-response surface are used to evaluate the data statistically and the volume of the peaks/depressions is calculated and used to quantify the volume of synergy/antagonism produced.

## Claims

1. Pharmaceutical composition comprising,
(i) a cytokine and,
(ii) a compound of the general formula (Ih) or pharmaceutically acceptable salts thereof with an acid or a base, or pharmaceutically acceptable prodrugs or a stereoisomer thereof, 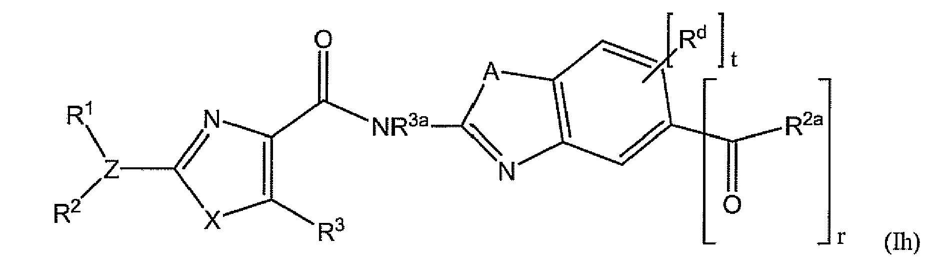
wherein
A is NR^{2'}, S or O;
t is 0 to 4;
r is 0, or 1;
R^{2a} is independently H, OH, SH, NH₂, alkyl, cycloalkyl, hydroxyalkyl, haloalkyl, haloalkyloxy, alkoxy, alkylamino, hydroxyalkylamino, halogen, aryl, or heteroaryl;
R^{3a} is H, OH, SH, NH₂, -C(NR⁷) NR^{7'} R⁸ -(CH₂)ₚaryl, -(CH₂)ₚNR⁷R⁸, -C(O)NR⁷R⁸, -N=CR⁷R⁸, -NR⁷C(O)R⁸, alkyl, cycloalkyl, hydroxyalkyl, haloalkyl, haloalkyloxy, alkoxy, alkylamino, hydroxyalkylamino, halogen, aryl, or heteroaryl;
R^{d} is H, halogen, alkyl, -C(NR⁷)NR^{7'}R⁸, -(CH₂)ₚaryl, -(CH₂)ₚNR⁷R⁸, - C(O)NR⁷R⁸, -N=CR⁷R⁸, -NR⁷C(O)R⁸, cycloalkyl, haloalkyl, hydroxyalkyl, hydroxyalkylamino, alkylamino, heteroaryl or aryl;
R¹ is -C(O)R^{7a} -C(O)CHR⁷R⁸, -C(O)NR⁷R⁸, -C(O)OR⁷, -R⁷C(O)R⁸, or -C (S)R^{7b};
R² is H, alkyl, cycloalkyl, heterocycloalkyl, haloalkyl, hydroxyalkyl, hydroxyalkylamino, alkylamino, or heteroaryl, or R¹ and R² together with the N-atom or the C-atom to which they are attached form a 3 to 8 membered saturated or at least partially unsaturated monocycloc or polycyclic ring system, wherein at least one or more of the carbon atoms in the ring is replaced by a heteroatom selected from O, N, and S and the ring can be substituted by one or more R⁹;
R³ is H, -C(O)NR^{a}R^{b}, halogen, alkyl, haloalkyl, aryl, heteroaryl, OH, SH, NR^{4'}OR^{5'}, NH₂, hydroxyalkylamino, alkylamino, alkoxy, cycloalkyl, hetero-cycloalkyl, hydroxyalkyl, or haloalkyloxy;
R^{a} is H, halogen, alkyl, -C(NR⁷)NR^{7'}R⁸, -(CH₂)ₚaryl, -(CH₂)ₚNR⁷R⁸, aryl, -C(O)NR⁷R⁸, -N=CR⁷R⁸, -NR⁷C(O)R⁸, cycloalkyl, heterocycloalkyl, haloalkyl, hydroxyalkyl, hydroxyalkyl amino, alkylamino, or heteroaryl;
R⁶ independently represents H, -CN, -OH, -SH, -CO₂R^{4'}, -C(O)R^{4'}, SO₂NR^{4'} , -NR^{4'}R^{5'}, -C(O)NR⁷R⁸, -SO₂-alkyl, -SO₂R^{4'}, SO₃R⁴', - N=CR^{4'}R^{5'}, -NR^{4'}C(O)R^{4"}, -NR^{4'}-CO-haloalkyl, -NO₂, -NR^{4'}-SO₂-haloalkyl, -NR^{4'}-SO₂-alkyl, -NR^{4'}-CO-alkyl, -NR^{4'}(CH₂)ₚheteroaryl, alkyl, cycloalkyl, alkylamino, alkoxy, alkylthio, halogen, haloalkyl, haloalkyloxy, -O(CH₂)ₚ[O(CH₂)ₚ]_{q}OCH₃, - C(NR^{4"})NR^{4'}benzimidazolyl, -C(NR^{4"})NR^{4'}-benzthiazolyl, C(NR^{4"})NR^{4'}benzoxazolyl, hydroxyalkyl, hydroxy-cycloalkyl, hydroxyalkylamino, heterocycloalkyl, aryl or heteroaryl;
R^{4'}, R^{4"}, R^{5'} independently are H, halogen, alkyl, -C(N-R⁷)NR^{7'}R⁸, -(CH₂)ₚaryl, haloalkyl, -(CH₂)ₚNR⁷R⁸, -C(O)NR⁷R⁸, -N=CR⁷R⁸, - NR⁷C(KO)R⁸, cycloalkyl, heterocycloalkyl, hydroxyalkyl, hydroxyalkylamino, alkylamino, heteroaryl, or aryl;
R⁷, R^{7'}, R⁸ independently are H, halogen, alkyl, cycloalkyl, heterocycloalkyl, haloalkyl, hydroxyalkyl, hydroxyalkylamino, alkylamino, arylamino heteroaryl, or aryl;
R^{7a} is cycloalkyl, haloalkyl, hydroxyalkyl, hydroxyalkylamino, heteroaryl, or aryl;
R^{7b} is H, halogen, alkyl, cycloalkyl, heterocycloalkyl, haloalkyl, hydroxyalkyl, hydroxyalkylamino, heteroaryl, or aryl;
X is NR^{2'}, O, or S;
Z is N or CR^{2'};
R^{2'} is H, alkyl, -C(O)NR⁷, -C(O)R^{b}, cycloalkyl, heterocycloalkyl, haloalkyl, hydroxyalkyl, hydroxyalkylamino, alkylamino, heteroaryl, or aryl;
p is 1 to 6;
q is 1 to 6;
R⁹ independently represents H, -CN, -OH, -SH, alkoxy, alkylthio, -CO₂R^{4'}, -C(O)R^{4a}, -C(O)NR⁴R⁸, -SO₂NR^{4'}, -NR^{4'}R^{5'}, -SO₂-alkyl, -SO₂R^{4'}, SO₃R^{4'}, -N=CR^{4'}R^{5'}, -NR^{4'}C(O)R^{4"}, -NR⁴'-CO-haloalkyl, -NO₂ -NR⁴'- SO₂-haloalkyl, -NR^{4'}-SO₂-alkyl, -NR⁴'-CO-alkyl, -NR^{4'}(CH₂)ₚheteroaryl, alkyl, hydroxyalkyl, cycloalkyl, halogen, haloalkyl, alkylamino, -O(CH₂)p[O(CH₂)ₚ]_{q}OCH₃, - C(NR^{4"})NR^{4'} benzimidazolyl, -C(NR^{4'})NR^{4'}-benzthiazolyl, -C(NR^{4"})NR^{4'}benzoxazolyl, hydroxycycloalkyl, hydroxy-alkylamino, haloalkyloxy, heterocycloalkyl, -(CH₂)ₚNR⁷COR⁸, aryl, or heteroaryl;
R^{4a} is H, C₁-C₆-alkyl, C₂-C₆-alkenyl, cycloalkyl, haloalkyl, hydroxyalkyl, hydroxyalkylamino, alkylamino, -C(NR⁷)NR^{7'}R⁸, -(CH₂)paryl, - (CH₂)pNR⁷,R⁸ -C(O)NR⁷R⁸, -N=CR⁷R⁸, -NR⁷C(O)R⁸, halogen, heteroaryl, or aryl;
wherein
a C₁-C₆-alkyl group, if not stated otherwise, denotes a linear or branched C₁-C₆-alkyl, which can optionally be substituted by one or more substituents R';
R' is independently H, -CO₂R", -CONHR", -CR"O, -SO₂NR", -NR"-CO-haloalkyl, -NO₂, -NR"-SO₂-haloalkyl -NR"-SO₂-alkyl -SO₂-alkyl, -NR"-CO-alkyl, -CN, alkyl, cycloalkyl, alkylamino, alkoxy, -OH, -SH, alkylthio, hydroxyalkyl, hydroxyalkylamino, halogen, haloalkyl, haloalkyloxy, aryl, or heteroaryl;
R" is independently H, haloalkyl, hydroxyalkyl, alkyl, cycloalkyl, aryl, or heteroaryl;
a C₂-C₆-alkenyl group, if not stated otherwise, denotes a linear or branched C₂-C₆-alkenyl ,
which can optionally be substituted by one or more substituents R'; an alkyl group, if not stated otherwise, denotes a linear or branched C₁-C₆-alkyl, a linear or branched C₂-C₆-alkenyl or a linear or branched C₂-C₆-alkynyl group, which can be substituted by one or more substituents R ; R being defined as above.
a cycloalkyl group denotes a non-aromatic ring system containing three to eight carbon atoms, wherein one or more of the carbon atoms in the ring can be substituted by one or more substituents R ; R being defined as above;
a heterocycloalkyl group denotes a non-aromatic ring system containing two to ten carbon atoms and at least one heteroatom selected from O, N, and S, wherein one or more of the carbon atoms in the ring can be substituted by R being as defined above;
an alkoxy group denotes an O-alkyl group, the alkyl group being as defined above;
an alkylthio group denotes an S-alkyl group, the alkyl group being as defined above;
a haloalkyl group denotes an alkyl group which is substituted by one to five halogen atoms, the alkyl group being as defined above;
a hydroxyalkyl group denotes an HO-alkyl group, the alkyl group being as defined above;
a haloalkyloxy group denotes an alkoxy group which is substituted by one to five halogen atoms, the alkyl group being as defined above;
a hydroxyalkylamino group denotes an (HO-alkyl)₂-N- group or HO-alkyl-NH-group, the alkyl group being as defined above;
an alkylamino group denotes an HN-alkyl or N-dialkyl group, the alkyl group being as defined above;
a halogen group is fluorine, chlorine, bromine, or iodine; an aryl group denotes an aromatic group having five to fifteen carbon atoms, which can be substituted by one or more substituents R', where R is as defined above;
an arylamino group denotes an HN-aryl or N-diaryl group, the aryl group being as defined above;
a heteroaryl group denotes a 5- to 10-membered aromatic heterocyclic group which contains at least one heteroatom selected from O, N, and S, wherein the heterocyclic group may be fused to another ring and the heterocyclic group or the fused ring can both be substituted
independently by one or more substituents R', wherein R is as defined above;

2. Composition according to claim 1, wherein
in formula (Ih), the following substituents are alone or in combination:
X is S or O, and,
R^{3a} is H.

3. Composition according to claim 1 or 2, wherein X is S.

4. Composition according to claim 1 to 3, wherein
Z is CR^{2'} and R¹ and R² together with the C-atom to which they are attached form a 6 membered saturated ring.

5. Composition according to claim 1 to 3, wherein
Z is N and R¹ and R² together with the N-atom to which they are attached form a 6 membered saturated ring.

6. Composition according to claims 1 to 5, wherein
R⁹ is is selected from the group of heteroaryl, aryl and benzyl.

7. Composition according to claim 6, wherein
R⁹ is selected from the group of thienopyrimidine, quinazoline, purine, pyrazolopyrimidine or triazolpyrimidine.

8. Composition according to one of the above claims, wherein
t is preferably 0 and,
r is preferably 1.

9. Composition according to one of the above claims, wherein
R^{2a} is selected from the group of OH, alkyl, aryl and heteroaryl.

10. Composition according to one of the above claims, wherein
the cytokine (i) is selected from the group of (a) an autocrine cytokine, (b) a paracrine cytokine and, (c) an endocrine cytokine.

11. Composition according to claim 10, wherein the cytokine is selected from the following group of cytokine families,
(i) the four α-helix bundle family, which consists of,
(a) the the IL-2 subfamily,
(b) the interferon (IFN) subfamily and,
(c) the IL-10 subfamily,
(ii) the IL-1 family,
(iii) the IL-17 family and,
(iv) chemokines.

12. Composition according to claims 10 and 11, wherein the cytokine is selected from the group of
(i) IL-2 family, including IL-4, IL-7, IL-9, IL-15 and IL-21,
(ii) the interferone family, including type I interferones, type II interferones and type III interferones and,
(iii) Tumor-Necrosis-Factor (TNF), including TNF-α (alpha) and TNF-β (beta).

13. Composition according to claim 12, wherein the cytokine is selected from the group of IFN-α (alpha), IFN-β (beta), IFN-γ (gamma), IL-2 and TNF.

14. Use of a composition according to claims 1 to 13 for the production of a medicament.
